(19) Europäisches Patentamt
European Patent Office
Office européen des brevets

(11) Veröffentlichungsnummer: **0 235 661**
**A2**

(12) # EUROPÄISCHE PATENTANMELDUNG

(21) Anmeldenummer: **87102177.0**

(51) Int. Cl.[4]: **C07F 9/65**, A61K 31/675

(22) Anmeldetag: **16.02.87**

(30) Priorität: **22.02.86 DE 3605847**
**23.04.86 DE 3613639**

(43) Veröffentlichungstag der Anmeldung:
**09.09.87 Patentblatt 87/37**

(84) Benannte Vertragsstaaten:
**AT BE CH DE ES FR GB GR IT LI LU NL SE**

(71) Anmelder: **Asta-Werke Aktiengesellschaft Chemische Fabrik**
**Artur-Ladebeck-Strasse 128 - 152 Postfach 14 01 29**
**D-4800 Bielefeld 14(DE)**

(72) Erfinder: **Hohorst, Hans-Jürgen, Prof. Dr. Dr.**
**Birkenweg 18**
**D-3550 Marburg-Marbach(DE)**
Erfinder: **Bielicki, Ludmilla, Dr.**
**Kennedyallee 62a**
**D-6000 Frankfurt 70(DE)**
Erfinder: **Voelcker, Georg, Dr.**
**Stifter Strasse 3**
**D-6369 Nidderau 1(DE)**
Erfinder: **Niemeyer, Ulf, Dr.**
**An den Gehren 30a**
**D-4800 Bielefeld 1(DE)**

(54) **Neue heterocyclische Verbindungen mit einem 2-[2-(N,N-Bis-(2-chlorethyl)-diamido-phosphoryl-oxy)ethyl]-Rest.**

(57) Die Erfindung betrifft Verbindungen der allgemeinen Formel

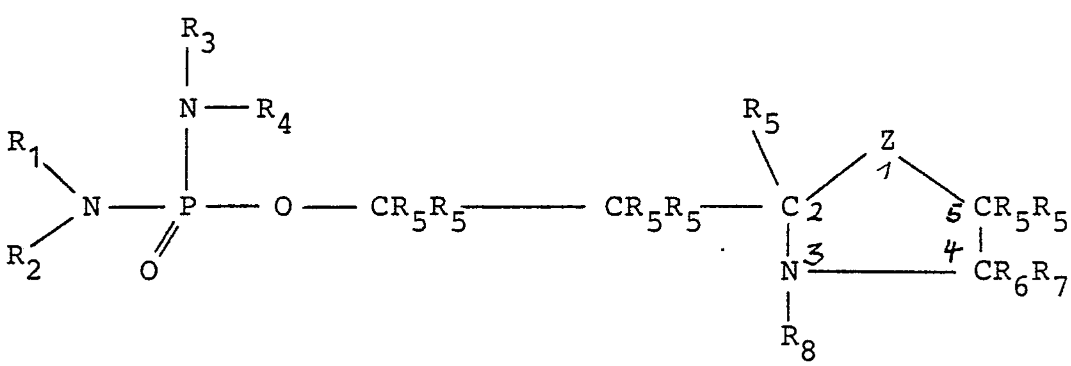

I

worin die Reste $R_1$, $R_2$, $R_3$ und $R_4$ Wasserstoff, $C_1$-$C_4$-Alkyl, 2-Chlorethyl, 2-Bromethyl oder 2-$C_1$-$C_4$-Alkansulfonyloxyethyl bedeuten, $R_8$ Wasserstoff, $C_1$-$C_6$-Alkyloder ein Acylrest ist, die Reste $R_6$ und $R_7$ Wasserstoff sind oder zusammen ein Sauerstoffatom bilden oder worin $R_6$ Wasserstoff ist und in diesem Falle $R_7$ eine Carboxylgruppe, eine $C_1$-$C_6$-Alkoxycarbonylgruppe, eine Aminocarbonylgruppe oder eine Alkylaminocarbonylgruppe ist, Z ein Schwefelatom, ein Sauerstoffatom, die Gruppe $>NR_5$, die Gruppe $-S-C(R_5)_2-$, $-O-C(R_5)_2-$oder $-NR_5-C(R_5)_2-$

EP 0 235 661 A2

bedeutet, und die Reste $R_5$ Wasserstoff oder $C_1$-$C_6$-Alkyl darstellen, sowie Verfahren zu deren Herstellung. Die Verbindungen der Formel I besitzen eine Antitumorwirkung.

# Neue heterocyclische Verbindungen mit einem 2-{2-[N,N-Bis-(2-chlorethyl)-diamido-phosphoryl-oxy]-ethyl}-Rest

Die Erfindung betrifft Verbindungen der Formel I gemäß Patentanspruch 1, Verfahren zu deren Herstellung sowie Arzneimittel, die als Wirkstoffe Verbindungen der Formel I enthalten.

Die erfindungsgemäßen Verbindungen stellen hochwirksame Cytostatika dar mit sehr geringer allgemeiner und lokaler Toxizität (zum Beispiel akute und subakte Toxizität). Insbesondere zeigen die erfindungsgemäßen Verbindungen auch eine höhere Selektivität der zytotoxischen Wirkung auf menschliche Tumorzellen bei geringerer Knochenmarkstoxizität (das heißt im Vergleich zur Knochenmarkstoxizität). Sie besitzen daher gegenüber dem bekannten Cytostatikum "Cyclophosphamid" beispielsweise eine erheblich größere therapeutische Breite (3-4 mal größer). Die erfindungsgemäßen Verbindungen sind beispielsweise auch lokal oder intracavitär wirksam.

Darüberhinaus sind die erfindungsgemäßen Verbindungen auch für die Behandlung von AIDS-Erkrankungen geeignet.

Die Antitumorwirkung der erfindungsgemäßen Verbindungen zeigt sich beispielsweise an folgenden Modellen: Blasensarkom von weiblichen Nacktmäusen (nu/nu-Mäuse); S180 Ascites-Tumor/Maus; Yoshida Ascites-Tumor/Ratte; P815Mastozytom der Maus; menschliche Tumorzellen im Clonogenic Assay in vitro.

Im Unterschied zum "Cyclophosphamid" und anderen cytostatisch wirksamen Oxazaphosphorinen erfolgt bei den erfindungsgemäßen Verbindungen die Freisetzung des Alkylans überraschenderweise viel langsamer.

Außerdem bewirken die erfindungsgemäßen Verbindungen eine Verstärkung der Immunabwehr (sie stellen daher biological response modifier dar). Diese Verstärkung der Immunabwehr erfolgt bei einer sehr niedrigen Dosierung (beispielsweise 0,05 -5 mg pro kg/Körpergewicht).

Falls in der Formel I mindestens zwei der Reste $R_1$, $R_2$, $R_3$ und $R_4$ 2-Chlorethyl, 2-Bromethyl oder 2-$C_1$-$C_4$-Alkansulfonyloxyethyl bedeuten, dann können auch in diesem Falle diese Reste jeweils gleich aber auch jeweils verschieden sein, das heißt beispielsweise kann $R_3$ 2-Chlorethyl und $R_1$ 2-Methansulfonyloxyethyl sein ($R_2$ und $R_4$ = Wasserstoff).

Falls die Reste $R_1$, $R_2$, $R_3$, $R_4$, $R_5$ und $R_8$ $C_1$-$C_6$-Alkyl bedeuten, handelt es sich vorzugsweise um Methylgruppen. In der Formel I vorkommende Alkyl-, Alkoxy-und Alkanoylgruppen können gerade oder verzweigt sein. Falls $R_8$ eine $C_2$-$C_6$-Alkanoylgruppe ist, handelt es sich insbesondere um Acetyl. Eine solche Alkanoylgruppe kann auch eine zusätzliche Carboxygruppe und/oder Aminogruppe enthalten, wobei die Carboxygruppe sich vorzugsweise am endständigen C-Atom der Alkanoylgruppe befindet ($\omega$-Stellung) und eine vorhandene Aminogruppe sich beispielsweise in Nachbarstellung zu der Carboxylgruppe oder in Nachbarstellung zu der CO-Gruppe befindet. Beispiele hierfür sind: $\alpha$ -oder $\gamma$-Glutamylrest. Falls $R_7$ und/oder $R_8$ $C_1$-$C_6$-Alkoxycarbonyl sind, besteht die Alkoxygruppe vorzugsweise aus einem oder zwei C-Atomen. Falls $R_7$ eine $C_1$-$C_6$-Alkylaminocarbonylgruppe ist, und diese eine zusätzliche Carboxylgruppe enthält, befindet sich die Carboxylgruppe vorzugsweise in der 1-Stellung des Alkylrestes. Alkylreste, die sich an einer Aminocarbonylgruppe der Formel I befinden, bestehen vorzugsweise aus einem oder zwei C-Atomen.

Besonders günstige Wirkungen zeigen Verbindungen der Formel I, worin Z ein Schwefelatom oder die Gruppe -S-C$(R_5)_2$-bedeutet ($R_5$ insbesondere Wasserstoff).

Falls $R_7$ eine Carbonsäureamidgruppe ist, und der Amidteil den Rest einer natürlichen Aminosäure oder eines natürlichen Dipeptids darstellt, ist diese natürliche Aminosäure beziehungsweise das Peptid über eine Aminogruppe beziehungsweise Aminofunktion der Aminosäure (vorzugsweise über die $\alpha$-ständige Aminogruppe, mit der Carbonsäuregruppe verknüpft. $R_7$ besitzt dann insbesondere die Struktur -CO-NH-CH($R_{18}$)-CO-$R_{19}$, worin $R_{19}$ OH, $C_1$-$C_6$-Alkoxy oder der Rest NH-CH($R_{18}$)-COR$_{20}$ ist und $R_{18}$ Wasserstoff, eine $C_1$-$C_{10}$-Alkylgruppe oder eine $C_1$-$C_{10}$-Alkylgruppe darstellt, welche durch eine Hydroxygruppe, eine $C_1$-$C_6$-Alkoxygruppe, eine Mercaptogruppe, eine $C_1$-$C_6$-Alkylthiogruppe, eine Phenylgruppe, eine Hydroxyphenylgruppe, eine Amino-$C_1$-$C_6$-alkylthiogruppe, eine Amino-$C_1$-$C_6$-alkyloxygruppe, eine Aminogruppe, eine Aminocarbonylgruppe, eine Ureidogruppe ($H_2$NCONH-), eine Guanidinogruppe, eine Carboxygruppe oder eine $C_1$-$C_6$-Alkoxycarbonylgruppe substituiert ist, oder $R_{18}$ bildet zusammen mit dem Strukturteil -NH-CH-CO-$R_{19}$ den 2-Carboxy-pyrrolidinyl-1-rest (Prolinyl-(1)-rest) oder den 4-Hydroxy-prolinyl-(1)-rest. $R_{20}$ bedeutet OH oder $C_1$-$C_6$-Alkoxy.

Falls $R_8$ der Rest einer natürlichen Aminosäure oder eines natürlichen Dipeptids ist, dann ist diese beziehungsweise das Peptid über die Carboxygruppe mit dem 3-ständigen Stickstoffatom des heterocyclischen Ringes der Formel I verknüpft. $R_8$ bedeutet dann insbesondere den Rest -CO-CH($R_{21}$)-NHR$_{22}$, worin $R_{21}$ Wasserstoff, eine $C_1$-$C_{10}$-Alkylgruppe oder eine $C_1$-$C_{10}$-Alkylgruppe darstellt, welche durch eine Hydroxy-

gruppe, eine $C_1$-$C_6$-Alkoxygruppe, eine Mercaptogruppe, eine $C_1$-$C_6$-Alkylthiogruppe, eine Phenylgruppe, eine Hydroxyphenylgruppe, eine Amino-$C_1$-$C_6$-alkylthiogruppe, eine Amino-$C_1$-$C_6$-alkyloxygruppe, eine Aminogruppe, eine Aminocarbonylgruppe, eine Ureidogruppe ($H_2NCONH$-), eine Guanidinogruppe, eine Carboxygruppe oder eine $C_1$-$C_6$-Alkoxycarbonylgruppe substituiert ist, oder worin $R_{21}$ den Rest $-CO-(CH_2)_n-CH(NHR_{22})-CO-OR_{23}$ darstellt, worin $R_{22}$ Wasserstoff, die Gruppe $-CO-CH(R_{21})NH_2$ oder die Gruppe $-CO-(CH_2)_n-CH(NH_2)-CO-OR_{23}$ ist und $R_{23}$ Wasserstoff oder $C_1$-$C_6$-Alkyl ist, und n die Zahlen 1, 2 oder 3 bedeutet.

Als Reste solcher natürlichen Aminosäuren beziehungsweise Dipeptide (Racemate, D-und L-Formen) kommen insbesondere die in Frage, die sich von folgenden Aminosäuren ableiten: Glycin, Prolin, Phenylalanin, Tyrosin.

Falls $R_7$ eine Carboxygruppe darstellt, können die erfindungsgemäßen Verbindungen der Formel I auch in Form der entsprechenden Salze mit physiologisch verträglichen anorganischen oder organischen Kationen vorliegen. Als anorganische Kationen kommen zum Beispeiel in Frage: $NH_4^+$, Kationen der Alkalimetalle (Na, K), der Erdalkalimetalle (Ca, Mg), des Wismuts, Aluminiums oder des Eisens. Als organische Kationen kommen zum Beispiel die Kationen folgender Amine in Frage:

Primäre, sekundäre oder tertiäre $C_1$-$C_6$-Alkylamine, die auch eine zweite Aminogruppe enthalten können - (wie zum Beispiel Ethylendiamin, N,N'-Dibenzylethylendiamin), Ethanolamine (Ethanolamin, 2-Dimethylaminoethanol, 2-Diethylaminoethanol, Cholin, Novocain), cyclische Amine (zum Beispiel $C_5$-$C_6$-Cycloalkylamine wie Cyclohexylamin), Guanidin, Piperidin, N-Ethyl-piperidin, Piperazin, Morpholin, N-Ethyl-morpholin, Nicotinamid, Glucosamin, Methylglucamin, Theobromin, Coffein, Purine, Imidazole. Weiterhin kommen die Kationen von Homocysteinthiolacton oder $\alpha$-Amino-$\epsilon$-caprolactam oder einer basischen Verbindung der Formel

$$R_{15} - \underset{\displaystyle NR_{16}R_{17}}{\underset{|}{CR_{14}}} - COR_{13} \qquad IV$$

in Frage. In der Formel IV bedeuten: $R_{13}$ eine Hydroxygruppe, eine Aminogruppe oder eine $C_1$-$C_6$-Alkoxygruppe, $R_{14}$ Wasserstoff oder eine Difluormethylgruppe, $R_{15}$ Wasserstoff, einen Indolyl-(3)-methylrest, Imidazolyl-(4)-methylrest, eine $C_1$-$C_{10}$-Alkylgruppe oder eine $C_1$-$C_{10}$-Alkylgruppe, die durch eine Hydroxygruppe, eine $C_1$-$C_6$-Alkoxygruppe, eine Mercaptogruppe, eine $C_1$-$C_6$-Alkylthiogruppe, eine Phenylgruppe, eine Hydroxyphenylgruppe, eine Amino-$C_1$-$C_6$-alkylthiogruppe, eine Amino-$C_1$-$C_6$-alkyloxygruppe, eine Aminogruppe, eine Aminocarbonylgruppe, eine Ureidogruppe ($H_2NCONH$-), eine Guanidinogruppe, eine Carboxygruppe oder eine $C_1$-$C_6$-Alkoxycarbonylgruppe substituiert ist, oder $R_{15}$ bildet zusammen mit dem Strukturteil $CR_{14}(NR_{16}R_{17})$ den Pyrrolidinyl-(2)-rest, den 4-Hydroxy-pyrrolidinyl-(2)-rest oder das 2-Oxo-3-amino-3-difluormethyl-piperidin und die Reste $R_{16}$ und $R_{17}$ sind Wasserstoff oder $C_1$-$C_6$-Alkylreste.

Insbesondere kommen als basische Salzkomponente Verbindungen der Formel IV in Frage, worin $R_{13}$ die Hydroxygruppe ist, die Reste $R_{14}$, $R_{16}$ und $R_{17}$ Wasserstoff sind und $R_{15}$ Wasserstoff oder eine $C_1$-$C_4$-Alkylgruppe bedeutet, welche auch durch eine Aminogruppe oder eine Guanidinogruppe substituiert sein kann (vorzugsweise in $\gamma$-oder $\delta$ -Stellung.

Die in der Formel IV vorkommenden Alkylgruppen, Alkoxygruppen, Alkylthiogruppen können gerade oder ver zweigt sein. Die $C_1$-$C_{10}$-Alkylgruppe enthält vorzugsweise 1-6 Kohlenstoffatome. Bei den Alkoxygruppen und Alkylthiogruppen handelt es sich vorzugsweise um solche mit 1-4, insbesondere 1-2 C-Atomen; dasselbe gilt hinsichtlich der Reste $R_{16}$ und $R_{17}$, falls diese Alkylreste sind. Falls in der Formel IV $R_{15}$ eine Alkylgruppe ist, welche eine Amino-$C_1$-$C_6$-alkylthio-($C_1$-$C_6$-alkoxy)gruppe enthält, dann handelt es sich vorzugsweise um folgende Gruppen: $H_2N-CH_2-CH_2-S-CH_2$-oder $H_2N-CH_2-CH_2-O-CH_2$-.

Bei den Verbindungen der Formel IV handelt es sich vorzugsweise um solche Verbindungen, wo $R_{13}$ eine Hydroxygruppe ist und die Reste $R_{14}$, $R_{16}$ und $R_{17}$ Wasserstoff sind und $R_{15}$ insbesondere die Bedeutungen annehmen kann, die hierfür im folgenden angegeben sind.

Bevorzugte basische Verbindungen der Formel IV sind beispielsweise solche, wo $R_{13}$ eine Hydroxygruppe, $R_{14}$ Wasserstoff oder Difluormethyl, $R_{15}$ eine $C_1$-$C_{10}$-Alkylgruppe, insbesondere $C_1$-$C_6$-Alkylgruppe, die (vorzugsweise in 2-, 3-, 4-, 5-oder 6-Stellung; Zählung beginnt stets an der Verknüpfungsstelle des Alkylrestes mit dem Restmolekül) eine Aminogruppe (insbesondere in 3-oder 4-Stellung), eine Amino-$C_2$-$C_4$-alkylthiogruppe, eine Amino-$C_2$-$C_4$-alkoxygruppe, einen Guanidinorest, einen Imidazolyl-(4)-methylrest oder einen Indolyl-(3)-methylrest enthält und $R_{16}$ und $R_{17}$ Wasserstoff oder $C_1$-$C_4$-Alkylreste bedeuten; oder Aminosäure-Derivate der Formel IV, wo $R_{13}$ eine Aminogruppe oder eine $C_1$-$C_4$-Alkoxygruppe, $R_{14}$ Wasser-

stoff, $R_{15}$ Wasserstoff, eine Phenylmethylgruppe, eine 4-Hydroxyphenylmethylgruppe oder eine $C_1$-$C_6$-Alkylgruppe, die (vorzugsweise in 2-, 3-, 4-, 5-oder 6-Stellung) eine Hydroxygruppe, eine Mercaptogruppe, eine $C_1$-$C_4$-Alkylthiogruppe, eine Aminocarbonylgruppe, eine $C_1$-$C_4$-Alkoxycarbonylgruppe oder Ureidogruppe enthält und $R_{16}$ und $R_{17}$ Wasserstoff oder $C_1$-$C_4$-Alkylreste bedeuten.

Bei den vorstehend genannten Aminosäuren beziehungsweise Aminosäurederivaten werden die Salze beispielsweise jeweils aus einem Mol der Verbindung I und einem Mol der Verbindung IV gebildet.

Weiterhin kommen als basische Verbindungen der Formel IV beispielsweise solche in Frage, wo $R_{13}$ eine Aminogruppe oder eine $C_1$-$C_4$-Alkoxygruppe ist, $R_{14}$ Wasserstoff oder Difluormethyl, $R_{15}$ eine $C_1$-$C_{10}$-Alkylgruppe, insbesondere $C_1$-$C_6$-Alkylgruppe, die (vorzugsweise in 2-, 3-, 4-oder $\omega$-Stellung) eine Aminogruppe, eine Amino-$C_2$-$C_4$-alkylthiogruppe, eine Amino-$C_2$-$C_4$-alkoxygruppe, einen Guanidinorest, einen Imidazolyl-(4)-methylrest oder einen Indolyl-(3)-methylrest enthält und $R_{16}$ und $R_{17}$ Wasserstoff oder $C_1$-$C_4$-Alkylreste bedeuten.

Bei den zuletzt genannten Aminosäurederivaten werden die Salze beispielsweise jeweils aus 2 Mol der Verbindung I und 1 Mol des Aminosäurederivats der Formel IV gebildet.

Einzelbeispiele für Verbindungen der Formel IV sind:

Asparaginsäurediamid (DL-Form), Asparaginsäurediethylester (L-Form), Citrullinamid ($H_2N$-CO-NH-$(CH_2)_3$-$CH(NH_2)$-$CONH_2$, L-Form), Ornithinethylester (L-Form), Arginin, Argininamid (L-Form), 4-Thialysin ($H_2N$-$CH_2$-$CH_2$-S-$CH_2$-$CH(NH_2)$-COOH), 2,6-Diamino-önanthsäure ($\epsilon$-Methyllysin), 4-Oxalysin ($H_2N$-$CH_2CH_2$-O-$CH_2$-CH-$(NH_2)$-COOH), Glycinamid, N,N-Dimethylglycinamid sowie der entsprechende Methyl-beziehungsweise Ethylester, Prolinamid, Hydroxyprolinamid, Phenylalaninamid, der Methyl-beziehungsweise Ethylester des Alanins oder des Phenylalanins, Homocysteinthiolacton (DL-Form), $\alpha$-Amino-$\epsilon$-caprolactam (D(+)-Form), Lysin (insbesondere L-Lysin), Difluormethyl-ornithin (DL-oder L-Form), Valinmethylester (L-,brm), Threoninethylester, Histidin, Histidinmethylester, Histidinamid, Alaninamid, Ornithin.

In den Salzen liegen für den Fall, daß in der Verbindung IV $R_{13}$ eine Aminogruppe oder eine Alkoxygruppe ist, und sonst eine basische Gruppe vorhanden ist oder für den Fall, daß $R_{13}$ die Hydroxygruppe ist und sonst aber zwei basische Gruppen vorhanden sind, Verbindung I (Säure-Komponente) und Verbindung II (basische Komponente) praktisch im Verhältnis 1:1 vor. (Dies gilt auch, falls die basische Komponente Homocysteinthiolacton oder $\alpha$-amino-$\epsilon$-caprolactam ist.) Ist andererseits in der basischen Komponente IV $R_{13}$ eine Aminogruppe oder eine Alkoxygruppe und enthält diese außer der $\alpha$-ständigen Aminogruppe noch eine weitere basische Gruppe, dann ist im allgemeinen das Verhältnis von Verbindung I zu Verbindung IV 2:1. Bei den erfindungsgemäßen Salzen handelt es sich um die neutralen Salze. Die pH-Werte solcher Salze liegen zum Beispiel zwischen 6 -9, insbesondere 6 -8.

Weiterhin kommen zum Beispiel als basische Salzkomponente in Frage: Amine der Formel NR'R"R‴, worin die Reste R', R" und R‴ gleich oder verschieden sind und Wasserstoff oder $C_1$-$C_4$-Alkylgruppen darstellen, die gegebenenfalls auch eine Hydroxygruppe enthalten können (zum Beispiel Oxyethyl).

Einzelbeispiele für solche Amine sind: Methylamin, Ethylamin, Propylamin, Isopropylamin, Dimethylamin, Diethylamin, Triethylamin, Trimethylamin. Tripropylamin, Methylethylamin, Dimethylethylamin, Diethylmethylamin, 2-Hydroxy-ethylamin, Bis-(2-hydroxy-ethyl)-amin, Tris-(2-hydroxy-ethyl)-amin, (2-Hydroxy-ethyl)-methylamin, (2-Hydroxy-ethyl)-dimethylamin, Bis-(2-hydroxy-ethyl)-methylamin, (2-Hydroxy-ethyl)-ethylamin, (2-Hydroxy-ethyl)-diethylamin, Bis-(2-hydroxy-ethyl)-ethylamin, (2-Hydroxy-ethyl)-methylethylamin. Als cyclische Amine kommen zum Beispiel in Frage: Morpholin, Piperidin, Pyrrolidin, Piperazin, als Diamine Ethylendiamin, Nicotinamid.

Die erfindungsgemäßen Verbindungen der Formel I können auch in Form von Salzen mit physiologisch verträglichen Säuren vorliegen. Als solche Säuren seien beispielsweise genannt: Halogenwasserstoffsäuren, Schwefelsäure, organische Mono-, Di-oder Tricarbonsäuren der aliphatischen, alicyclischen, aromatischen oder heterocyclischen Reihe sowie Sulfonsäuren.

Beispiele hierfür sind: Ameisen-, Essig-, Propion-, Bernstein-, Glykol-, Milch-, Äpfel-, Wein-, Zitronen-, Ascorbin-, Malein-, Fumar-, Hydroxymalein-, oder Brenztraubensäure; Phenylessig-, Benzoe-, p-Aminobenzoe-, Anthranil-, p-Hydroxy-benzoe-, Salicyl-oder p-Aminosalicylsäure, Embonsäure, Methansulfon-, Äthansulfon-, Hydroxyäthansulfon-, Äthylensulfonsäure; Halogenbenzolsulfon-, Toluolsulfon-, Naphthalinsulfonsäure oder Sulfanilsäure oder auch 8-Chlor-theophyllin.

Je nach den Verfahrensbedingungen und Ausgangsstoffen erhält man die Endstoffe der Formel I in freier Form (als basische Verbindung), als Säure oder in Form ihrer Salze. Die Salze der Endstoffe können in an sich bekannter Weise beispielsweise mit Alkali, Säuren oder Ionenaustauschern wieder in die Basen beziehungsweise Verbindungen I mit freier Carboxylgruppe übergeführt werden. Von den letzteren lassen sich durch Umsetzung mit organischen oder anorganischen Säuren oder entsprechenden basischen Verbindungen, insbesondere solchen, die zur Bildung von therapeutisch verwendbaren Salzen geeignet sind, Salze gewinnen.

So werden beispielsweise die Carbonsäuren bei 0 -20° C in wäßriger Lösung mit einer Base, vorzugsweise Natriumhydrogencarbonat neutralisiert und die Lösung gefriergetrocknet.
Aminosäuresalze, zum Beispiel Lysin-und Argininsalze, lassen sich durch Zugabe der äquivalenten Mengen der freien Aminosäure zu wässrigen Lösungen von Verbindungen der Formel I bei einem Ausgangs-pH von 4 -5 und Raumtemperatur und anschließende Lyophilisierung gewinnen.

Der Austausch der basischen Komponente eines Salzes der Verbindung I gegen eine basische Komponente kann aber auch beispielsweise an sauren Ionenaustauschern erfolgen, die mit einer basischen Verbindung IV beladen sind. Als saure Ionenaustauscher kommen zum Beispiel solche in Frage, deren polymere Matrix Sulfonsäure-Gruppen oder Carbonsäure-Gruppen tragen. Die Matrix der Ionenaustauscher kann zum Beispiel aus einem Polystyrolharz, gegebenenfalls mit einem Gehalt von 2 bis 16, vorzugsweise 4 bis 8 an Divinylbenzol oder auch einem Phenolharz bestehen. Der Polystyrolionenaustauscher ist vorzugsweise gelförmig. Die Beladung des Ionenaustauschers mit der basischen Komponente kann beispielsweise auf folgende Weise erfolgen: 150 ml Ionenaustauscherharz 1,2 mval/ml * in einer Säule - (Durchmesser ca. 4 cm) mit Kühlmantel werden mit Salzsäure regeneriert, mit destilliertem Wasser neutral und chloridionenfrei gewaschen, mit einer 10 %igen wässrigen Lösung der basischen Verbindung (220 mmol) IV behandelt und mit destilliertem Wasser neutral gewaschen.

Zu dem Verfahren:

Das Verfahren wird in einem Lösungsmittel bei Temperaturen zwischen -70 und 100° C, vorzugsweise - 10 bis 80° C, insbesondere 5 bis 60° C durchgeführt, das heißt gegebenenfalls unter Kühlen, bei Raumtemperatur oder unter Erwärmen. Falls X und Y der Ausgangsverbindung II Alkoxygruppen sind oder zusammen eine Alkylendioxygruppe bilden, erfolgt die Umsetzung mit der anderen Ausgangskomponente III zweckmäßig in Anwesenheit eines sauren Katalysators, wie einer anorganischen oder organischen Säure, wie zum Beispiel Trichloressigsäure, p-Toluolsulfonsäure, Ameisensäure, Salzsäure, Trifluormethansulfonsäure. Wenn X und $R_4$ den 6-Ring (Oxazaphosphorinanring) bilden und Y Hydroxy, Alkoxy, Alkylthio oder die Gruppe $-S-alk-SO_3Z$ ist, erfolgt die Einstellung des für die Reaktion günstigen pH-Bereichs, beispielsweise pH 5 -12, insbesondere 7 -9, zum Beispiel mittels Alkalilaugen (NaOH). Als Lösungsmittel kommen zum Beispiel in Betracht: Wasser, Alkohole, insbesondere Alkanole mit 1-6 C-Atomen wie Methanol, Ethanol, n-Propanol, Isopropanol oder Isobutanol, Alkylketone mit jeweils 1-4 C-Atomen wie insbesondere Aceton, Methylethylketon, aprotische Lösungsmittel (zum Beispiel Dimethylsulfoxid, Acetonitril, N-Methylpyrrolidon, Dimethylformamid, Hexamethylphosphorsäuretriamid), halogenierte Kohlenwasserstoffe mit 1-3 C-Atomen wie Chloroform, Ethylendichlorid, gesättigte cyclische Ether wie Tetrahydrofuran, Dioxan, gesättigte niedere aliphatische Ether wie Diethylether oder ähnliche Lösungsmittel beziehungsweise Gemische aus mehreren solcher Lösungsmittel.

Die Einführung des Restes $R_8$ durch Acylierung gemäß dem Herstellungsverfahren erfolgt durch Isocyansäure, $C_1$-$C_6$-Alkylisocyansäure oder durch eine Säure der Formel R-OH, wobei R eine $C_1$-$C_6$-Alkoxycarbonylgruppe, eine Aminocarbonylgruppe, eine $C_1$-$C_6$-Alkylaminocarbonylgruppe, eine Di-$C_1$-$C_6$-alkylaminocarbonylgruppe oder eine $C_2$-$C_6$-Alkanoylgruppe oder der Rest einer natürlichen Aminosäure beziehungsweise eines natürlichen Dipeptids ist und eine solche Säure beziehungsweise das Dipeptid vorzugsweise aktiviert ist. Falls für die Acylierung eine solche aktivierte Säure verwendet wird, handelt es sich vorzugsweise um Verbindungen der Formel R-W, worin R die oben angegebenen Bedeutungen hat und W Halogen (zum Beispiel Chlor oder Brom) ist, aber auch zum Beispiel eine Gruppe der Formel -OR', -SR' oder eine Gruppe der Formel $-OSO_3H$ oder -OCO-R" bedeutet und R' einen $C_1$-$C_6$-Alkylrest oder auch einen Phenylrest, einen durch Nitrogruppen, $C_1$-$C_4$-Alkoxygruppen, $C_1$-$C_4$-Alkylgruppen oder Halogenatome (Chlor, Fluor, Brom) substituierten Phenylrest, einen Cyanmethylrest oder einen Carboxymethylrest und R" einen geraden oder verzweigten $C_1$-$C_6$-Alkylrest, einen $C_1$-$C_6$-Alkoxyrest, einen Phenoxyrest oder einen Benzyloxyrest darstellt, wobei R auch die Gruppe COCl sein kann, falls W Halogen ist und sich in diesem Falle eine anschließende Umsetzung in üblicher Weise mit $NH_3$, einem $C_1$-$C_6$-Alkylamin oder einem Di-$C_1$-$C_6$-alkylamin anschließt. Falls W ein Halogenatom bedeutet, handelt es sich vorzugsweise um Chlor, Brom oder Jod; falls R' beziehungsweise R" Alkylreste, Halogenalkylrest oder Alkoxyreste bedeuten, dann sind diese vorzugsweise niedermolekular und bestehen aus 1 -4 Kohlenwasserstoffatomen. Häufig, insbesondere wenn W ein Halogenatom oder die Gruppe -OCOR" bedeutet, ist die Gegenwart eines säurebindenden

* Die Hersteller der Ionenaustauscher geben die Kapazität der Ionenaustauscher (das heißt die Menge der funktionellen Gruppen wie $-SO_3H$, $-CO_2H$ in mval/ml oder mval/g des Ionenaustauscherharzes an.

Stoffes wie Alkalihydroxiden, Alkalicarbonaten, Alkali hydrogencarbonaten, Alkaliacetaten, Erdalkalicarbonaten, tertiären Aminen (Trialkylamine wie Triethylamin, Pyridin) oder auch Alkalihydriden und ähnlichen zweckmäßig. Dabei kann das säurebindende Agens auch gleichzeitig allein oder im Gemisch mit anderen üblichen Mitteln als Lösungsmittel benutzt werden (zum Beispiel Pyridin).

Falls die freie Säure R-OH verwendet wird, so ist deren Aktivierung durch die Gegenwart von Kondensationsmitteln wie Dicyclohexylcarbodiimid, Tetraäthylpyrophosphit, 5-(3'-Sulfonphenyl)-äthylisooxazol, Schwefligsäure-bisalkylamiden (zum Beispiel $SO[N(CH_3)_2]_2$), N,N'Carbonyldiimidazol und so weiter erforderlich (Organic Reactions, Vol. 12, 1962, Seiten 205 und 239).

Falls $R_8$ eine $C_1$-$C_6$-Alkylgruppe ist, kann diese in solche Verbindungen der Formel I, worin $R_8$ Wasserstoff ist, durch Alkylierung eingeführt werden. Ebenso kann in Verbindungen der Formel I, worin Z die Gruppe NH oder -NH-$C(R_5)_2$-ist, der Wasserstoff dieses basischen Stickstoffatoms mittels Alkylierung durch eine $C_1$-$C_6$-Alkylgruppe ersetzt werden.

Diese Alkylierung erfolgt beispielsweise durch Umsetzung mit Verbindungen der Formel $C_1$-$C_6$-Alkyl-Hal $ArSO_2O$-$C_1$-$C_6$-Alkyl und $SO_2(O$-$C_1$-$C_6$-Alkyl$)_2$, wobei Hal ein Halogenatom (insbesondere Chlor, Brom oder Jod) und Ar ein aromatischer Rest (zum Beispiel ein gegebenenfalls durch einen oder mehrere niedere Alkylreste substituierter Phenyl-oder Naphthylrest sein kann. Beispiele sind p-Toluolsulfonsäure-$C_1$-$C_6$-alkylester, $C_1$-$C_6$-Dialkylsulfate, $C_1$-$C_6$-Alkylhalogenide und ähnliche. Die Alkylierungsreaktion wird gegebenenfalls zweckmäßig in Gegenwart von üblichen säurebindenden Mitteln durchgeführt. Als säurebindende Mittel kommen beispielsweise die gleichen in Frage wie für die Acylierung.

Die Acylierung beziehungsweise die Alkylierung wird zum Beispiel bei Temperaturen zwischen -60 und 220° C, vorzugsweise -20 und 150° C, insbesondere + 20° C und 110° C in inerten Lösungsmitteln oder Suspensionsmitteln durchgeführt. Als Lösungs-oder Dispergiermittel kommen beispielsweise in Betracht: aromatische Kohlenwasserstoffe wie zum Beispiel Benzol, Toluol, Xylol; aliphatische Ketone wie zum Beispiel Aceton, Methylethylketon; halogenierte Kohlenwasserstoffe, wie zum Beispiel Chloroform, Tetrachlorkohlenstoff, Chlorbenzol, Methylenchlorid; aliphatische Ether, wie zum Beispiel Butylether; cyclische Ether, wie zum Beispiel Tetrahydrofuran, Dioxan; Sulfoxide, wie zum Beispiel Dimethylsulfoxid; tertiäre Säureamide, wie zum Beispiel Dimethylformamid, N-Methylpyrrolidon, Hexamethylphosphorsäuretriamid; aliphatische Alkohole wie Methanol, Ethanol, Isopropanol, Amylalkohol, tert.-Butanol, cycloaliphatische Kohlenwasserstoffe, wie Cyclohexan und ähnliche. Auch wässrige Mischungen der genannten Lösungsmittel können verwendet werden. Häufig arbeitet man bei der Rückflußtemperatur der verwendeten Lösungs-beziehungsweise Dispergiermittel. Häufig werden die Alkylierungsreaktionskomponenten im Überschuß eingesetzt. Die Alkylierung kann auch in Gegenwart von Tetraalkylammoniumsalzen (insbesondere der Halogenide) in Kombination mit Alkalihydroxiden bei Temperaturen zwischen 0 - 100° C, vorzugsweise 20 -80° C in einem aprotischen Lösungsmittel oder auch in Chloroform oder Methylenchlorid vorgenommen werden. Als aprotische Lösungsmittel kommen insbesondere in Betracht: tertiäre Amide (Dimethylformamid, N-Methyl-Pyrrolidon, Hexamethylphosphorsäuretriamid), Dimethylsulfoxid, Acetonitril, Dimethyloxyethan, Aceton, Tetrahydrofuran. Bei der Acylierung und Alkylierung kann man auch so vorgehen, daß man erst von der umzusetzenden Verbindung eine Alkaliverbindung herstellt, indem man sie in einem inerten Lösungsmittel wie Dioxan, Dimethylformamid, Benzol oder Toluol mit einem Alkalimetall, Alkalihydriden oder Alkaliamiden (insbesondere Natrium oder Natriumverbindungen) oder Butyllithium bei Temperaturen zwischen 0 und 150° C umsetzt und zum Beispiel dann das acylierende beziehungsweise alkylierende Agens (Verbindung R-W, W = Halogen) zufügt.

Anstelle der angeführten Alkylierungs-und Acylierungsmittel können auch andere in der Chemie gebräuchliche chemisch-äquivalente Mittel verwendet werden (siehe zum Beispiel auch L.F. und Mary Fieser "Reagents for Organic Synthesis", John Wiley and Sons, Inc., New York, 1967, Vol. 1, Seiten 1303-4 und Vol, 2, Seite 471).

Die Acylgruppen in den Verbindungen der Formel I können solvolytisch wieder abgespalten werden, wodurch die entsprechenden Verbindungen der Formel I erhalten werden, worin $R_8$ Wasserstoff ist. Diese solvolytische Abspaltung erfolgt beispielsweise durch Verseifung mit verdünnten Säuren oder mittels basischer Substanzen (Pottasche, Soda, wässrige Alkalilösungen, $NH_3$) bei Temperaturen zwischen 10 und 150° C, insbesondere 20 -100° C.

Die Überführung des Restes $R_7$ in andere hierfür mögliche Bedeutungen kann durch folgende Reaktionen erfolgen:

a) Verseifung

Die Verseifung besteht in einer Überführung von Verbindungen der Formel I, worin $R_7$ eine $C_1$-$C_6$-Alkoxycarbonylgruppe, eine Aminocarbonylgruppe oder eine Alkylamino-oder Dialkylaminocarbonylgruppe - (jeweils mit Alkylresten aus 1 -6 C-Atomen) ist, in eine Verbindung der Formel I, worin $R_1$ die Carboxylgruppe ist. Diese Verseifung erfolgt beispielsweise durch Wasser, verdünnte wässrige Alkalilaugen, alkoholische Alkalilaugen oder auch gegebenenfalls mittels Mineralsäuren wie Salzsäure oder Schwefelsäure in alkoholischer oder wässrig-alkoholischer Lösung bei Temperaturen zwischen 20 und 200° C, vorzugsweise 20 -100° C oder auch mittels NaCl/Dimethylsulfoxid. Es können auch weitere inerte Lösungsmittel, wie Dioxan, Tetrahydrofuran, Dimethylformamid, Dimethylacetamid verwendet werden.

b) Veresterung

Die Veresterung kann beispielsweise dadurch erfolgen, daß carbonsaure Salze von Verbindungen der Formel I ($R_7$ = $CO_2H$), insbesondere Alkalisalze oder Silbersalze, mit $C_1$-$C_6$-Alkalihalogeniden (Chloriden, Bromiden, Jodiden) umgesetzt werden oder durch Behandeln der freien Säuren mit $C_1$-$C_6$-Diazoparaffinen oder $C_1$-$C_6$-aliphatischen Alkoholen in Gegenwart von sauren Stoffen, wie Salz-oder Schwefelsäure, Chlorsulfonsäure, aromatischen Sulfonsäuren (p-Toluolsulfonsäure) oder komplexbildenden Stoffen wie Bortrifluorid oder Zinkchlorid oder indem die entsprechenden Säurehalogenide der Formel I $R_7$ = COHal; Hal = Cl, Br, J) oder Säureanhydride der Formel I ($R_7$ = $C_2$-$C_6$-Alkanoyloxycarbonylgruppe) auf $C_1$-$C_6$-Alkohole, gegebenenfalls in Gegenwart basischer Stoffe wie Pyridin, Triäthylamin, Alkali-oder Erdalkalimetallhydroxiden, -alkoholaten, -carbonaten oder -acetaten zur Einwirkung kommen. Diese Veresterung wird bei Temperaturen zwischen 20 bis 250° C, insbesondere 60 bis 100° C, mit oder ohne Lösungsmittel durchgeführt. Als Lösungsmittel kommen beispielsweise in Betracht: Aromatische Kohlenwasserstoffe, wie Benzol, Toluol, niedere aliphatische Alkohole, Dimethylformamid, cyclische Äther, Dimethylsulfoxid, N-Methyl-pyrrolidon, Sulfolan, Chloroform, Tetramethylharnstoff.

Die oben erwähnten Halogenide ($R_7$ = COHal) können zum Beispiel durch Umsetzung mit Halogenierungsmitteln, wie Phosphortri-und -pentahalogenide (Chloride, Bromide, Jodide), Thionylhalogenide (Chlorid, Bromid, Jodid), Phosphoroxyhalogenide (Phosphoroxychlorid, Phosphoroxybromid), Sulfurylhalogenide - (Sulfurylchlorid, Sulfurylbromid, Sulfuryljodid) oder Phosgen erhalten werden. Die Reaktion kann mit oder ohne Lösungsmittel bei Temperaturen zwischen 20 -150° C, vorzugsweise 30 -100° C, durchgeführt werden. Als Lösungsmittel kommen inerte Mittel, wie Dioxan, Tetrahydrofuran, aromatische Kohlenwasserstoffe (Benzol, Toluol), Halogenkohlenwasserstoffe (Chloroform, Methylenchlorid) in Betracht. Häufig ist es zweckmäßig, die entsprechenden Ausgangssäuren ($R_7$ = $CO_2H$) in Form ihrer Alkalisalze (Natrium, Kaliumsalze oder auch Silbersalze) einzusetzen.

c) Die Herstellung der Amide erfolgt durch Umsetzung von Verbindungen der Formel I, worin $R_7$ eine Carboxylgruppe oder eine $C_1$-$C_6$-Alkoxycarbonylgruppe ist (gegebenenfalls über die entsprechenden Halogenide oder Anhydride) mit Ammoniak, $C_1$-$C_6$-Alkylaminen, Dialkylaminen mit Alkylresten aus 1 bis 6 C-Atomen, oder den entsprechenden Aminosäuren beziehungsweise Dipeptiden beziehungsweise entsprechend geschützten Aminosäuren beziehungsweise geschützten Dipeptiden nach den üblichen Methoden der Peptidchemie. Die Umsetzung wird zum Beispiel in einem üblichen Lösungs-oder Suspensionsmittel durchgeführt, wobei als Lösungsmittel die bereits genannten in Betracht kommen. Auch Wasser kann verwendet werden. Falls als Ausgangsstoffe Verbindungen der Formel I eingesetzt werden, worin $R_7$ die Gruppe -COHal bedeutet, ist die Gegenwart säurebindender Stoffe (Alkalihydroxide, Alkalicarbonate, Alkalihydrogencarbonate, Alkaliacetate, Erdalkalicarbonate, Trialkylamine, Pyridin und ähnliche) oder auch überschüssiges Ammoniak beziehungsweise Amin zweckmäßig. Dabei kann das säurebindende Agens auch gleichzeitig allein oder im Gemisch mit anderen üblichen Mitteln als Lösungsmittel benutzt werden (zum Beispiel Pyridin). Falls als Ausgangssubstanz eine Verbindung der Formel I eingesetzt wird, worin $R_7$ die Carboxylgruppe ist, so ist deren Aktivierung in Gegenwart von Kondensationsmitteln wie Dicyclohexylcarbodiimid, Schwefligsäure-bis-alkylamiden (zum Beispiel $SO[N(CH_3)_2]_2$), N,N'-Carbonyldiimidazol und ähnlichen erforderlich (Organic Reactions Vol. 12, 1962, Seiten 205 und 239).

Erfolgt die Herstellung der Amide über die Anhydride, so können letztere zum Beispiel durch Umsetzung der Salze (Alkalisalze, Silbersalze) von Verbindungen der Formel I, worin $R_7$ die Carboxylgruppe ist, mit den entsprechenden $C_2$-$C_6$-Alkanoylhalogeniden (Chloriden, Bromiden, Jodiden) oder durch Umsetzung von Verbindungen der Formel I, worin $R_7$ -COCl, -COBr oder -COJ ist, mit Alkalisalzen beziehungsweise Silbersalzen der entsprechenden $C_2$-$C_6$-Alkancarbonsäuren erhalten werden. Diese Umsetzungen werden zum Beispiel in einem Lösungs-oder Suspensionsmittel bei Temperaturen zwischen 20 -250° C durchgeführt.

Falls die Ausgangsverbindung der Formel III eine Carboxygruppe enthält, kann diese auch in Form eines entsprechenden üblichen Salzes eingesetzt werden. Gegebenenfalls ist in dem Reaktionsprodukt das Kation dann in bekannter Weise gegen ein physiologisch verträgliches Kation auszutauschen.

Eine Ausgangsverbindung, worin X zusammen mit $R_4$ eine einfache Bindung darstellt und Y eine Hydroxygruppe ist (zum Beispiel 4-Hydroxy-cyclophosphamid), kann auch in situ aus der entsprechenden Verbindung, worin Y Wasserstoff ist (zum Beispiel Cyclophosphamid) durch bekannte Ozonisierung, zum Beispiel in Wasser, wäßrigem Tetrahydrofuran oder wäßrigem Alkohol, hergestellt werden und dann ohne Isolierung direkt weiter mit einer Verbindung III umgesetzt werden. Die Ozonisierung wird zweckmäßig in Abwesenheit von Wasserstoffperoxid durchgeführt.

Die Isolierung der Verfahrensprodukte erfolgt vorzugsweise durch chromatographische Verfahren, insbesondere durch Chromatographie an Silicagel oder Sephadex (siehe Beispiel 1c).

Die Verfahrensprodukte können durch Beimengung von Thiolen mit freier SH-Gruppe, wie zum Beispiel Cystein, Cysteamin, 2-Mercaptoethanol stabilisiert werden. Dies erfolgt beispielsweise, indem man die Verbindungen der Formel I in einer wässrigen Lösung der Thiol-Verbindung löst, und diese Mischung dann einer Gefriertrocknung unterwirft. Für eine solche Stabilisierung werden zum Beispiel pro 1 Mol Verbindung I 0,02 -0,05 Mol der thiol-Verbindung verwendet.

Die Ausgangsstoffe der Formel II, worin X und Y je eine Alkoxygruppe oder einen Alkylendioxyring darstellen, sind zum Teil beispielsweise aus der japanischen Patentanmeldung 71/25140 (japanische Publikationsschrift 4738928) bekannt beziehungsweise können analog der dort angegebenen Methode beziehungsweise wie folgt erhalten werden: Umsetzung von Phosphoroxychlorid mit einem entsprechenden Acetal der Formel $HO-C(R_5)_2-C(R_5)_2-CR_5XY$ (X, Y = $C_1$-$C_6$-Alkoxy oder $O-(CH_2)_n-O$ mit n = 1 -5) in einem inerten Mittel bei tiefer Temperatur (zum Beispiel -20 bis +10° C) in Gegenwart eines tertiären Amins und anschließende Umsetzung des so erhaltenen Reaktionsproduktes mit den Aminen $HNR_1R_2$, $HNR_3R_4$ beziehungsweise Ammoniak in einem oder zwei Schritten, gegebenenfalls in Gegenwart eines tertiären Amins bei Temperaturen zwischen -20 und 15° C. Analog lassen sich die Ausgangsstoffe der Formel II, worin X und Y je eine Alkylthiogruppe oder zusammen einen Alkylendithioring bedeuten, durch Umsetzung von Phosphoroxytrichlorid mit den entsprechenden Thioacetalen der Formel $HO-C(R_5)_2-C(R_5)_2-CR_5XY$ (X, Y = $C_1$-$C_6$-Alkylthio oder $-S-(CH_2)_n-S$ mit n = 1-5), und anschließend mit den Aminen $HNR_1R_2$, $HNR_3R_4$ beziehungsweise Ammoniak in einem Schritt oder zwei Schritten herstellen.

Ausgangsstoffe der Formel II, worin X und Y zusammen ein Sauerstoffatom darstellen, können beispielsweise aus den entsprechenden Hydroxyverbindungen (X = OH, Y = H) in an sich bekannter Weise durch Oxydation erhalten werden (zum Beispiel analog D. Swern, J. Org. Chem. 43, 1978, Seite 2480; A. Myles et al, Tetrahedron Letters, 1977, Seite 2475; D. Swern, Synthesis, 1981, Seite 165; G. Piancatelli et al, Synthesis, 1982, Seite 245).
Weiterhin können solche Ausgangsstoffe aus den entsprechenden Thioacetalen (X und Y = Alkylthio oder $S-(CH_2)_n-S$) in an sich bekannter Weise durch Thioether-Spaltung gewonnen werden (zum Beispiel analog E.J. Corey, J. Org. Chem. 36, 3553 (1971) oder aus den entsprechenden Oximen (X und Y = =N-OH) in an sich bekannter Weise durch Oxim-Spaltung (zum Beispiel analog J.H. Boyer, Chem. Rev. 80, 541 - (1980), P.J. Mattingly et al, J. Org. Chem. 45, 410 (1980) und Synthesis 1976, 808).

Nicht bekannte Ausgangsverbindungen der Formel III können nach bekannten Verfahren hergestellt werden. Beispielsweise werden die Ester der Carbonsäuren der Formel III durch Umsetzung der Carbonsäuren mit dem entsprechenden Alkohol in salzsaurem Medium erhalten. N-Acetyl-Verbindungen werden durch Einwirkung von Acetylchlorid oder Acetanhydrid auf die Amino-Verbindung gewonnen. Beispielsweise wird zur Darstellung von N-Acetyl-Cystein das Cystein mit Acetylchlorid in N,N'-Diacetyl-Cystin umgewandelt und das Reaktionsgemisch mit Zink in Essigsäure bei 50 -60° C reduziert. N-Alkyl-Verbindungen werden beispielsweise durch Reduktion entsprechender Thiazolidin-Verbindungen erhalten. So wird N-Methyl-Cystein durch Einwirkung von Natrium auf Thiazolidin-4-carbonsäure in flüssigem Ammoniak und Hydrolyse des Reaktionsproduktes mit Säure erhalten.

Unter den Verfahrensprodukten der Formel I werden alle möglichen Stereoisomere und Mischungen davon verstanden. Im einzelnen handelt es sich beispielsweise um Verbindungen, die am Phosphoratom oder an einem C-Atom des Heterocycluses unterschiedliche Konfigurationen aufweisen.

Diastereomere Gemische können in bekannter Weise beispielsweise durch fraktionierte Kristallisation oder durch analytische und präparative Hochdruckflüssigkeitschromatographie getrennt werden. Die reinen Enantiomere können nach den üblichen Methoden der Racematspaltung beispielsweise durch fraktionierte Kristallisation von diastereomeren Salzen erhalten werden.

Zur Racematspaltung kommen beispielsweise als optisch aktive Basen zum Beispiel 1-Phenylethylamin, Brucin, Chinidin, Strychnin und Cinchonin sowie weitere Basen und Methoden in Frage, die in "Optical Resolution Procedures for Chemical Compounds", Vol. 2, Paul Newman, 1981, Verlag Optical Resolution Information Center in Riverdale, USA, beschrieben sind. Hierzu wandelt man beispielsweise ein erfindungsgemäß racemisches Salz auf die bereits angegebene Weise in ein Salz mit einer der vorstehend genannten optisch aktiven Basen um, trennt die Enantiomeren in bekannter Weise.

Die erfindungsgemäßen Verbindungen zeigen bei der NMRI-Maus (weiblich) mit S-180 Ascites-Tumor nach einmaliger intraperitionealer Gabe eine gute kurative Wirkung. Beispielsweise wird bei obengenannter Versuchsmethode bei einer Dosierung von 100 mg/kg Maus mit der Verbindung gemäß Beispiel 1 eine völlige Heilung bei 5 von 5 therapierten Tieren erzielt. Die niedrigste, bereits kurativ wirksame Dosis in dem oben genannten Tierversuch liegt bei 3 mg/kg intraperitoneal.

Als allgemeiner Dosisbereich für die kurative Wirkung in obigem Tierversuch kommt beispielsweise eine Dosierung von 10 mg/kg -200 mg/kg intraperitoneal, insbesondere 100 mg/kg in Frage.

Beim P 815 Mastozytom * Ascites-Tumor bei der $BDF_1$-Maus (männlich) zeigen die erfindungsgemäßen Verbindungen beispielsweise bei einer Dosis von 1000 mg/kg (intraperitoneal) Körpergewicht eine Lebenszeitverlängerung (ILS) von 136 % und mehr. Die niedrigste, bereits wirksame Dosis in diesem Versuch ist 100 mg/kg intraperitioneal. Als allgemeiner Dosisbereich für die Heilwirkung beim P 815 Ascites-Tumor kommt beispielsweise in Frage: 100 -1500 mg/kg intraperitoneal oder intravenös, insbesondere 1000 mg/kg.

Die Wirkungsrichtung der erfindungsgemäßen Verbindungen ist mit der Wirkung des bekannten Arzneimittels Cyclophosphamid oder Ifosfamid vergleichbar. Jedoch besteht hierzu insbesondere folgender Unterschied: Deutlich verringerte Allgemeintoxizität, verringerte Knochenmarkstoxizität und fehlende Toxizität in den Harn abführenden Wegen und der Harnblase. Außerdem sind die erfindungsgemäßen Verbindungen im Gegensatz zu Cyclophosphamid oder Ifosfamid auch bei lokaler oder intracavitärer Applikation wirksam.

Beispiel für die zytostatische Wirkung von 2-(2-[N,N-Bis (2-Chlorethyl)-diamido-phosphoryl-oxy]ethyl)-homothiazolidin-4-carbonsäure auf den P815 Mastozytom-Tumor der Maus:
Weiblichen $BDF_1$-Mäusen wurden $2 \times 10^6$ P815 Mastozytomzellen intraperitoneal injiziert. Die Kontrolltiere - (ohne Zytostatikum) starben nach 6,4 Tagen (Mittel von 10 Tieren).
Die behandelten Tiere erhielten am 3. Tag nach Tumortransplantation steigende Dosen der erfindungsgemäßen Substanz:

| Dosis mg/kg | ILS %<br>nach 28 Tagen | Heilungsrate %<br>nach 40 Tagen |
|---|---|---|
| 110 | 206 | 0 |
| 681 | 282 | 0 |
| 1000 | 352 | 30 |
| 1470 | 375 | 20 |

ILS = Verlängerung der Lebensspanne gegenüber der unbehandelten Kontrollgruppe (Increase of life span over untreated control).
Zahl der behandelten Tiere: 10/Dosis.

Nach 40 Tagen Beobachtungszeit waren bei der Dosis 1000 mg/kg 30 % (3/10) der Tiere endgültig geheilt. Im Vergleich dazu waren mit einer gleich toxischen Dosis von Cyclophosphamid nach 28 Tagen Beobachtungszeit alle behandelten Tiere gestorben.

Nach fraktionierter Gabe von jeweils 110 mg/kg der erfindungsgemäßen Substanz, täglich für 6 Tage intraperitoneal injiziert, waren beispielsweise nach 4 Wochen Beobachtungszeit noch 50 % der behandelten Tiere am Leben und sind demnach endgültig geheilt.

Als Indikationen für die erfindungsgemäßen Verbindungen kommen außer allen Formen von Krebserkrankungen auch die Therapie von AIDS in Betracht.

Die akute Toxizität der erfindungsgemäßen Verbindungen an der Maus (ausgedrückt durch die $LD_{50}$ mg/kg: Methode nach Miller und Tainter: Proc. Soc. Exper. Biol. a Med. 57 (1944) 261) liegt beispielsweise bei intravenöser Applikation zwischen 1000 und 3000 mg/kg (meist oberhalb von 1500 mg/kg).

Die pharmazeutischen Zubereitungen enthalten im allgemeinen zwischen 50 bis 1500, vorzugsweise 150 bis 500 mg der erfindungsgemäßen aktiven Komponenten.

*geschwulstartige Wucherung atypischer Gewebsmastzellen

Die Verabreichung kann beispielsweise in Form von Tabletten, Kapseln, Pillen, Dragees, Zäpfchen, Salben, Gelees, Cremes, Puder, Stäubepulver, Aerosolen oder in flüssiger Form erfolgen. Als flüssige Anwendungsformen kommen zum Beispiel in Frage: Ölige oder alkoholische beziehungsweise wässrige Lösungen sowie Suspensionen und Emulsionen. Bevorzugte Anwendungsformen sind Tabletten, die zwischen 50 und 200 mg oder Lösungen, die zwischen 2 bis 10 % an aktiver Substanz enthalten.

Die Einzeldosis der erfindungsgemäßen aktiven Komponenten kann beispielsweise liegen

a) bei oralen Arzneiformen zwischen 0,5 bis 7 g, vorzugsweise 1 bis 3 g,

b) bei parenteralen Arzeiformen (zum Beispiel intravenös, intramuskulär) zwischen 100 mg bis 5 g, vorzugsweise 0,5 bis 2 g,

c) bei Arzneiformen zur Inhalation (Lösungen oder Aerosole) zwischen 0,5 bis 2 g, vorzugsweise 1 g,

d) bei Arzneiformen zur rektalen oder vaginalen Applikation zwischen 0,5 bis 5 g, vorzugsweise 2 g,

e) bei Arzneiformen zur lokalen Applikation auf die Haut und Schleimhäute (zum Beispiel in Form von Lösungen, Lotionen, Emulsionen, Salben und so weiter) zwischen 0,2 bis 1,8 g, vorzugsweise 1 g.

-(Die Dosen sind jeweils bezogen auf die freie Base) -

Beispielsweise können 3 mal täglich 1 bis 10 Tabletten mit einem Gehalt von 150 bis 700 mg wirksamer Substanz oder zum Beispiel bei intravenöser Injektion 1 bis 3 mal täglich eine Ampulle von 2 bis 10 ml Inhalt mit 50 bis 2000 mg Substanz empfohlen werden. Bei oraler Verabreichung ist die minimale tägliche Dosis beispielsweise 0,5 mg; die maximale tägliche Dosis bei oraler Verabreichung soll nicht über 8 g liegen.

Für die Behandlung von Hunden und Katzen liegt die orale Einzeldosis im allgemeinen zwischen ungefähr 50 und 300 mg/kg Körpergewicht; die parenterale Dosis ungefähr zwischen 15 und 250 mg/kg Körpergewicht.

Für die Behandlung von Pferden und Vieh liegt die orale Einzeldosis im allgemeinen zwischen ungefähr 50 und 200 mg/kg; die parenterale Einzeldosis ungefähr zwischen 20 und 200 mg/kg Körpergewicht.

Die akute Toxizität der erfindungsgemäßen Verbindungen an der Maus (ausgedrückt durch die LD 50 mg/kg; Methode nach Miller und Tainter: Proc. Soc. Exper. Biol. a. Med. 57 (1944) 261) liegt beispielsweise bei oraler Applikation zwischen 1800 und 2400 mg/kg (beziehungsweise oberhalb 1800 mg/kg).

Die Arzneimittel können in der Humanmedizin, der Veterinärmedizin sowie in der Landwirtschaft allein oder im Gemisch mit anderen pharmakologisch aktiven Stoffen verwendet werden.

Weiterhin sind die erfindungsgemäßen Verbindungen auch für die Behandlung von AIDS-Erkrankungen geeignet.

Es hat sich gezeigt, daß die erfindungsgemäßen Verbindungen der Formel I aufgrund ihrer Strukturanalogie zum physiologischen Substrat als hochspezifische Hemmstoffe des für die Proliferation von lymphoiden Zellen (insbesondere von T-Zellen) verantwortlichen Enzyms wirken.

Damit kommen die erfindungsgemäßen Verbindungen der Formel I auch für die Therapie von AIDS und seinen Manifestationen (zum Beispiel Kaposisyndrom und ähnliche) in Betracht: Da die Vermehrung des HTLV-III-Virus (AIDS-Virus) an die Gegenwart und Vermehrung spezifischer lymphoider Zellen, in diesem Fall zum Beispiel $T_4$ Helferzellen, gebunden ist, können Verbindungen der Formel I durch selektive Vernichtung der T-Lymphozyten im Wirtsorganismus gegebenenfalls kombiniert mit einer geeigneten Immuntherapie und gegebenenfalls in Kombination mit Knochenmarktransplantationen oder adoptiver Immuntherapie durch Transfer von Interleukin-2 stimulierten T-Zell-Subpopulationen (deren Bildung durch Interleukin-2 stimuliert wurde) den Vermehrungszyklus des AIDS-Virus unterbrechen und so die Krankheit heilen.

Die Verbindungen der Formel I können gegen AIDS bereits in niedriger Dosierung wirken, beispielsweise in Dosierungen von 0,05 -5 mg/kg Körpergewicht.

Insbesondere in frühen Stadien der Erkrankung sowie im Anschluß an eine zytostatische Therapie können Verbindungen der Formel I durch spezifische Hemmung von T-Suppressorlymphozyten das bei AIDS stark verringerte Verhältnis T-Helferlymphozyten / T-Suppressorlymphozyten vergrößern und so die Immunabwehr stimulieren.

Beispiel 1

2-{2-[N,N-Bis(2-chlorethyl)-diamido-phosphoryl-oxy]ethyl}-thiazolidin-4-carbonsäure

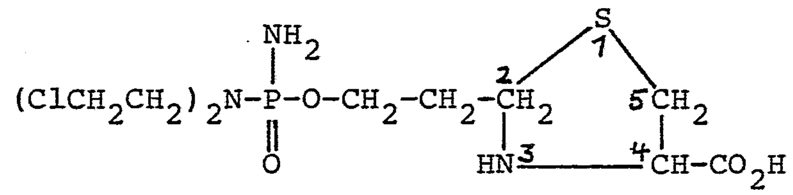

a) Herstellung aus einer Verbindung II, worin X zusammen mit $R_4$ eine einfache Bindung darstellt und Y eine Hydroxygruppe ist:

5,0 g (18 mmol) 4-Hydroxy-cyclophosphamid und 2,4 g (20 mmol) L-Cystein in 12 ml destilliertem Wasser werden mit 20 ml 1 N Natronlauge versetzt. Das Reaktionsgemisch wird 3 Stunden unter $N_2$-Atmosphäre gerührt, unterhalb von 0° C im Hochvakuum eingeengt, der Rückstand mit Methanol/Dichlormethan (3:2) angerieben, der unlösliche Anteil abgesaugt und die Lösung mehrfach an Silicagel mit Methanol/Dichlormethan (3:2) eluiert.
Die reine Fraktion mit $R_f$ = 0,38 (freie Carbonsäure) wurde im Vakuum eingeengt, der Rückstand 2 mal mit trockenem Ether hehandelt und abschließend zu einem glasigen Pulver im Hochvakuum getrocknet.
Ausbeute: 4,0 g (58 % der Theorie).
Das Natriumsalz kann zum Beispiel wie folgt erhalten werden: 1 g der Carbonsäure D 18 038 (Chiffre-Bezeichnung für die freie Säure gemäß obiger Formel) werden in wenig Wasser aufgenommen, mit der äquimolaren Menge Natriumhydrogencarbonat versetzt und zu einem Pulver gefriergetrocknet. Ausbeute: 1,1 g (100 % der Theorie).
Wenn nichts anderes angegeben ist, erfolgt die Bestimmung der $R_f$-Werte in einer Trogkammer mit Kammersättigung bei Zimmertemperatur:
Stationäre Phase: Aluminiumfolie, die mit Cellulose beschichtet ist; die Cellulose enthält einen Fluoreszenzindikator.

Aufgetragene Substanzmenge: ca. 5 $\mu$l einer Lösung von 10 mg/ml in Methanol.

Laufmittel: Wasser-Acetonitril 1:2

Laufstrecke des Lösungsmittels: 10 cm

Die Identifizierung erfolgte durch die folgenden speziellen Anfärbereagenzien: 4-(4-Nitro-benzyl)-pyridin - (Reagenz auf alkylierende Gruppen) und Ninhydrin-Sprühtest.

b) Herstellung aus einer Verbindung II, worin X zusammen mit $R_4$ eine einfache Bindung darstellt und Y eine $C_2$-Alkoxygruppe (Ethoxygruppe) ist:

Zu einer Lösung von 4,6 g (15 mmol) 4-Ethoxy-cyclophosphamid in 10 ml Ethanol wird eine Lösung von 1,9 g (16,5 mmol) L-Cystein in 24 ml destilliertem Wasser und 16 ml 1 N Natronlauge gegeben. Nach 3 Stunden wird aufgearbeitet wie vorstehend angegeben ist.
Ausbeute: 2,8 g (48 % der Theorie)
$R_f$ = 0,38 (freie Säure).

c) Herstellung aus einer Verbindung II, worin X und Y jeweils eine Ethoxygruppe sind:

1,05 g (3 mmol) 0-(3,3-Diethoxy-propyl)-[N,N-bis-(2-chlorethyl)]-phosphorsäure-diamid werden in einer Lösung von 544 mg (4,5 mmol) L-Cystein in ca. 20 ml 0,01 N HCl suspendiert und auf 60° C erwärmt, bis eine klare Lösung entstanden ist (etwa 75 Minuten). Nach dem Einstellen auf pH 7 mit NaOH wird die Reaktionslösung durch Gelfiltration über eine Sephadex G-10 Säule mit 0,07 M Phosphatpuffer als Laufmittel getrennt (Sephadex ist ein Medium aus modifiziertem Dextran zur Gelfiltration in wässrigem und polarem Medium).
Die das Reaktionsprodukt enthaltenden Fraktionen werden gesammelt und ebenfalls durch Trennung über eine Sephadex G-10 Säule mit Laufmittel $H_2O$ entsalzt. Man erhält so eine wässrige Lösung von chromatographisch reinem Reaktionsprodukt D 18 038 die, bei -20° C eingefroren, mehrere Tage haltbar ist.

Die Konzentration des Alkylans in der Lösung wird mit dem NBP-Test (NBP = 4-(4-Nitro-benzyl)-pyridin) bestimmt und liegt bei durchschnittlich 5 mg/ml, was einer Gesamtmenge von ca. 400 mg Reaktionsprodukt D 18 038 pro Ansatz entspricht (Ausbeute 35% der Theorie).

Eine andere Arbeitsweise mit anschließender Stabilisierung durch Beimengung von Cystein ist die folgende:

3 mmol (1050 mg) O-(3,3-Diethoxy-propyl)-[N,N-bis-(2-chlorethyl)]-phosphorsäure-diamid (Aldophosphamiddiethylacetal) werden zusammen mit 4,5 mmol (544 mg) L-Cystein in 25 ml 0,01 N Ameisensäure gelöst. Nach 55 Minuten bei 57° C wird dreimal mit 25 ml Chloroform ausgeschüttelt. Die Chloroformreste werden im Wasserstrahlvakuum (15 Minuten) entfernt. Die wässrige Lösung wird eingefroren und über Nacht gefriergetrocknet. Man erhält ca. 1 g Lyophilisat, das mit 60 ml Peroxid freiem Tetrahydrofuran (Reinigung über eine Aluminiumoxid-Säule unmittelbar vor Gebrauch) bei 4° C (Kühlraum) extrahiert wird (1 Minute schütteln, anschließend 1 Minute Ultraschallbad).

Nach Zentrifugation (Sorvall RC5C Zentrifuge, 40 000 g, 15 Minuten 4° C) und Einengen des Tetrahydrofuran-Überstandes bis zur Trockene wird der Rückstand in einer 0,32 mg/ml wässrigen L-Cystein-Lösung gelöst, wobei soviel wässrige L-Cystein-Lösung verwendet wird, daß die Konzentration an Verbindung I 20 mg pro ml ist, und diese Lösung gefriergetrocknet.

Die so hergestellten Präparationen enthalten 5 Mol% L-Cystein.

Ausbeute ca. 200 mg Substanz D 18 038 (Ausbeute ca. 18 % bezogen auf das eingesetzte Aldophosphamiddiethylacetal).

Das nach den Methoden a) -c) erhaltene Reaktionsprodukt fällt als Diastereomerengemisch an.

Das O-(3,3-Diethoxy-propyl)-[N,N-bis-(2-chlorethyl)] phosphorsäure-diamid, welches gemäß der Verfahrens weise c) als Ausgangssubstanz verwendet wird, kann beispielsweise wie folgt erhalten werden:

3,6 g (23 mmol) Phosphoroxychlorid in 25 ml trockenem Methylenchlorid werden tropfenweise bei 0 bis 5° C innerhalb von 2 Stunden unter Rühren mit einer Lösung von 3,4 g (23 mmol) 3-Hydroxypropanal-Diethylacetal in 10 ml Methylenchlorid und 3,3 ml Triethylamin versetzt. Es wird bei 0° C eine Stunde nachgerührt. Anschließend werden 4,2 g Bis-(2-chlorethyl)-amin-Hydrochlorid zugegeben und bei 5 -10° C 7 ml Triethylamin in 10 ml Methylenchlorid zugetropft. Das Reaktionsgemisch wird eine Stunde bei 5° C gerührt und bei 4° C stehengelassen. Am nächsten Tag werden 2,8 g flüssiger Ammoniak in 5 ml Methylenchlorid unter Rühren eingetropft. Die Temperatur läßt man auf 20° C ansteigen, rührt 3 Stunden nach, wäscht mit Wasser, trocknet die organische Phase über Natriumsulfat und engt ein. Der Rückstand wird an Silicagel mit Hilfe von Chloroform/Methanol (10:1) chromatographiert. Ausbeute: 3,9 g; $R_f$-Wert: 0,6; (Laufmittel: Benzol/Chloroform/Methanol = 2:1:1, Kammersättigung, Laufhöhe 15 cm, Anfärbung: Jod-Dampf, Entwicklung bei Zimmertemperatur).

Herstellung des Lysinsalzes:

Zur Salzbildung wird die Menge L-Lysin zugegeben, welche der Konzentration der Substanz D 18 038 äquimolar ist, und die Lösung im Gefriertrockner lyophilisiert. Das erhaltene farblose Lyophilisat ist stark hygrospkopisch, läßt sich aber, im Gegensatz zur freien Substanz D 18 038, unter Feuchtigkeitsausschluß bei +4° C über Wochen unverändert aufbewahren.

Das Lysinsalz enthält noch geringe Mengen des Lösungsmittels (Wasser) und liegt als ein Diastereomerengemisch vor.

Dünnschichtchromatographie des Lysinsalzes (bei der Dünnschichtchromatographie wird das Salz in L-Lysin und die Substanz D 18 038 getrennt, daher werden bei dieser Methode die $R_f$-Werte dieser beiden Komponenten erhalten): Alufolie beschichtet mit Cellulose (0,1 mm Schichtdicke);

Laufmittel Wasser / Acetonitril 1:2

Substanz D 18 038: $R_f$ = 0,68 L-Lysin: $R_f$ = 0,08

Stabilität:

Das Lysinsalz zeigt auch nach wochenlanger Lagerung bei -20° C keine Veränderungen im Chromatogramm.

Herstellung von Injektionslösungen:

Lösungen von 2-{2-[N,N-Bis-(2-chlorethyl)-diamido-phosphoryl-oxy]ethyl}-thiazolidin-4-carbonsäure werden unmittelbar vor Gebrauch durch 45 minütige Inkubation von 4-Hydroxy-cyclophosphamid mit einem 10 %igen Überschuß von L-Cystein in 0,07 M Phosphatpuffer pH 7 bei 37° C hergestellt.

Beispiel 2

2-{2-[N,N-Bis-(2-chlorethyl)-diamido-phosphoryl-oxy]ethyl}-thiazolidin-4-carbonsäureethylester

a) Herstellung aus einer Verbindung II, worin X zusammen mit $R_4$ eine einfache Bindung darstellt und Y eine Hydroxygruppe ist:
5,0 g (18 mmol) 4-Hydroxycyclophosphamid und 3,4 g (18 mmol) L-Cystein-ethylester-Hydrochlorid werden in 18 ml Wasser und 18 ml 1 N Natronlauge 4 Stunden unter Stickstoff-Atmosphäre gerührt. Die Reaktionslösung wird mit Dichlormethan ausgeschüttelt und die organische Phase mit wenig 0,1 N Schwefelsäure und 3 mal mit Wasser gewaschen, über Natriumsulfat getrocknet und eingeengt. Der Rückstand wird mit trockenem Ether behandelt und im Hochvakuum zu einem dünnschichtchromatographisch einheitlichen Öl konzentriert.
$R_f$-Wert = 0,83 (Bedingungen wie Beispiel 1)
Ausbeute: 4,8 g (65 % der Theorie).

b) Herstellung aus einer Verbindung, worin X und Y jeweils eine Ethoxygruppe sind:

10 mg 0-(3,3-Diethoxy-propyl)-[N,N-bis(2-chlor-ethyl)]-phosphorsäure-diamid und 8 mg Cysteinäthylesterhydrochlorid werden in 1 ml 0,01 HCl auf 56° C erhitzt.
Nach 60 Minuten ist das Acetal vollständig verschwunden und im Dünnschichtchromatogramm ein neuer Peak ($R_f$-Wert: 0,83) entstanden. Die Aufarbeitung der Reaktionslösung erfolgt wie unter a) angegeben ist.

Das nach a) oder b) erhaltene Reaktionsprodukt besteht jeweils aus einem Diastereomerengemisch.

Beispiel 3

3N-Acetyl-2-{2-[N,N-Bis-(2-chlorethyl)-diamido-phosphoryl-oxy]ethyl}-thiazolidin-4-carbonsäure

10 mg 0-(3,3-Diethoxy-propyl)-[N,N-bis(2-chlor-ethyl)]-phosphorsäure-diamid und 7,5 mg L-N-Acetylcystein werden in 0,01 N HCl auf 56° C erhitzt. Es wird mit Methylenchlorid ausgeschüttelt, die Methylenchloridphase mit Natriumsulfat getrocknet, das Methylenchlorid entfernt, der Rückstand in Methanol aufgenommen und über eine Sephadex Säule LH 20 mit Methanol als Eluent chromatographiert (Sephadex LH 20 ist ein mit Epichlorhydrin dreidimensional vernetztes Dextran mit Hydroxypropylgruppen, das für die Gelfiltration in organischen Lösungsmitteln verwendet wird).

Beispiel 4

2-{2-[N,N-Bis-(2-chlorethyl)-diamido-phosphoryl-oxy]ethyl}-thiazolidin-4-carbonsäure-methylester

1 g (~3 mmol) 0-(3,3-Diethoxy-propyl)-[N,N-bis-(2-chlorethyl)]-phosphorsäure-diamid und 770 mg (~4,5 mmol) L-Cysteinmethylesterhydrochlorid werden in 20 ml 0,01 N HCl 75 Minuten auf 56° C erhitzt. Anschließend wird die Reaktionslösung mit NaOH neutralisiert. Dann wird mit 3 × 50 ml Methylenchlorid ausgeschüttelt und die vereinigte organische Phase über Natriumsulfat getrocknet. Nach Einengen der organischen Phase wird das gelbe Öl in Wasser/Methanol 1:1 aufgenommen und über eine Sephadex G-10 Säule mit 0,07 M Phosphatpuffer pH 7 getrennt.
Das Eluat, welches das Reaktionsprodukt enthält, wird mit 3 × 250 ml Methylenchlorid ausgeschüttelt und die Methylenchloridphase eingeengt. Der Rückstand wird in Methanol aufgenommen und über Sephadex LH20 mit Methanol als Eluent chromatographiert. Die das Reaktionsprodukt enthaltende Fraktion wird eingeengt und im Hochvakuum getrocknet. Man erhält ein chromatographisch reines gelbliches Öl, das sich gut in polaren organischen Lösungsmitteln löst, weniger gut in apolaren Lösungsmitteln und Wasser. Das

Reaktionsprodukt ist ein Diasteromerengemisch.
$R_f$ = 0,7
Ausbeute: 200 mg (17 % der Theorie)

Beispiel 5

2-{2-[N,N-Bis-(2-chlorethyl)-diamido-phosphoryl-oxy]ethyl}-4-oxo-thiazolidin

1 g (~3 mmol) 0-(3,3-Diethoxy-propyl)-[N,N-bis-(2-chlorethyl)]-phosphorsäure-diamid und 730 mg (4,5 mmol) N-(2-Mercapto-propionyl)-glycin werden in 20 ml 0,01 N HCl 60 Minuten auf 56° C erwärmt. Dann wird mit 3 x 50 ml Methylenchlorid ausgeschüttelt und die vereinigte organische Phase über Natriumsulfat getrocknet. Nach Einengen der organischen Phase wird der Rückstand in Methanol aufgenommen und über eine Sephadex LH20 Säule mit Methanol chromatographiert. Man erhält eine Fraktion, welche zwei neu entstandene stark UV-aktive Peaks enthält. Nach Einengen des Eluats erhält man ein gelbes Öl, das in Wasser und apolaren Lösungsmitteln schlecht löslich ist. Das Reaktionsprodukt ist ein Diastereomerengemisch.
$R_f$ = 0,75
Ausbeute: 230 mg (19 % der Theorie).

Beispiel 6

3N-L-γ-Glutamyl-2-{2-[N,N-bis(2-chlorethyl)-diamido-phosphoryl-oxy]ethyl}-thiazolidin-4-carbonsäure-(carboxymethylamid)

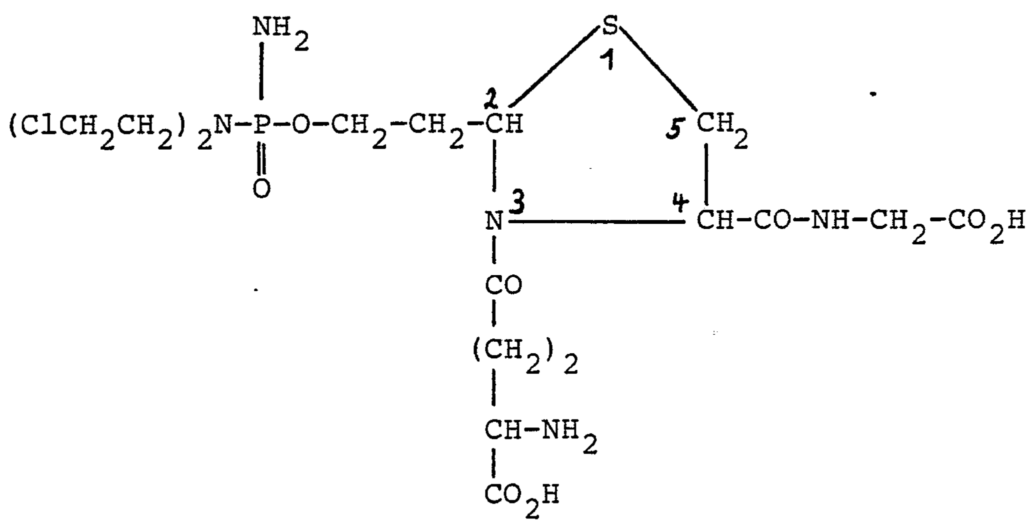

3 mmol (1,09 g) 0-(3,3-Diethoxy-propyl)-[N,N-bis-(2-chlorethyl)-phosphorsäurediamid werden mit 4,5 mmol (1,4 g) L-Glutathion in 25 ml 0.01 N Ameisensäure 60 Minuten auf 56° C erhitzt. Anschließend wird das Reaktionsgemisch mit 3 x 25 ml Chloroform extrahiert und lyophilisiert. Die Trennung erfolgt über eine Kieselgel-Säule, wobei das Kieselgel mit Octadecanyl-Gruppen versetzt ist ($C_{18}$-Reversed Phase -Säule), wobei zuerst das Wasser mit Glutathion abgetrennt wird. Anschließend wird mit Methanol eluiert und man erhält das Glutathion-Thiazolidon-Derivat.
Nach Entfernen des Methanols wird das Reaktionsprodukt aus Wasser/Ethanol 1:1 ausgefällt: Ausbeute: ca. 200 mg; $R_f$-Wert: 0,48.

Beispiel 7

2-{1,1-Dimethyl-1-[N,N-bis(2-chlorethyl)-diamido-phosphoryl-oxy]ethyl} -thiazolidin-4-carbonsäure

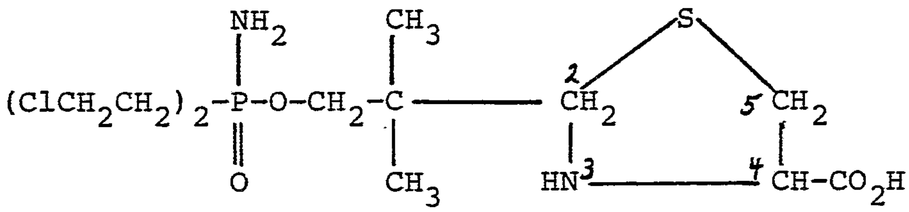

In einer Lösung von 1,6 g L-Cystein (13 mmol) in ca. 50 ml 0,01 N Ameisensäure werden 3 g O-[(3,3-Diethoxy-2,2-dimethyl-propyl-(1)]-N,N-bis-2-chlorethyl)]-phosphorsäurediamid suspendiert (8,5 mmol). Die Inkubationszeit beträgt 30 Minuten bei 80° C. Danach wird 3 mal mit 50 ml Chloroform ausgeschüttelt, die wässrige Phase im Vakuum von Chloroformrückständen befreit und gefriergetrocknet.

Es bleibt ein farbloser Feststoff zurück, der im wesentlichen nur noch das Thiazolidin-Reaktionsprodukt und L-Cystein enthält. Zur Isolierung des Verfahrensproduktes wird das Lyophilisat 2 mal mit je 30 ml absolutem Tetrahydrofuran digeriert. Man erhält aus dem Tetrahydrofuran-Extrakt 1,35 g relative sauberes Reaktionsprodukt (> 95 %), entsprechend 38 % der theoretischen Ausbeute. Zur endgültigen Reinigung kann die Substanz auf eine "Reverse Phase" -Säule aufgetragen werden (zum Beispiel Kieselgel, welches mit Octadecanyl-Gruppen vernetzt ist). Die Säule läßt sich mit $H_2O$ spülen, ohne daß das Thiazolidin-Reaktionsprodukt heruntergewaschen wird. Das saubere Produkt kann dann mit Methanol-Wasser - (mindestens 20 % Methanol-Anteil) oder mit reinem Methanol isoliert werden. Die analytischen Daten des so gereinigten Verfahrensproduktes sind:

Elementaranalyse

| | C | H | N |
|---|---|---|---|
| berechnet | 35,29 | 5,88 | 10,29 |
| gefunden | 34,99 | 5,74 | 10,01 |

Hochdruckflüssigkeitschromatographie (HPLC)Säule $C_{18}$ Novo-Pak
Laufmittel Methanol/0,02 N Phosphatpuffer pH 7 35:65
Ein Peak nach ca. 15 Minuten.

Dünnschichtchromatogramm Cellulose auf Aluminiumfolie
Laufmittel: Acetonitril/$H_2O$ 2:1
$R_f$ = 0,89.

Beispiel 8

2-{2-[N,N-Bis(2-chlorethyl)-diamido-phosphoryl-oxy]ethyl}-homothiazolidin-4-carbonsäure

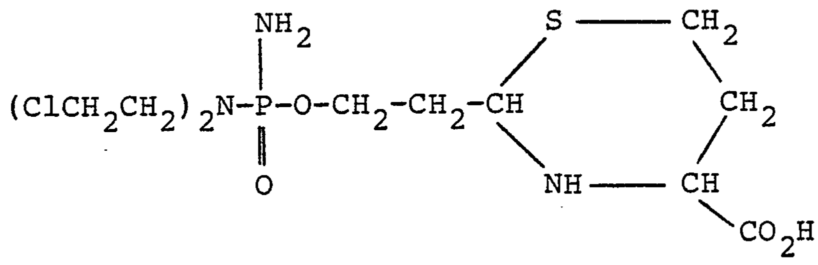

1.05 g (3 mmol) O-(3,3-Diethoxy-propyl)-[N,N-bis-(2-chlor-ethyl)]-phosphorsäure-diamid und 4,5 mmol - (0,61 g) DL-Homocystein werden in 25 ml 0,01 N Ameisensäure 60 Minuten lang auf 56° C erhitzt. Dann wird das Reaktionsgemisch lyophilisiert und das Lyophilisat in 25 ml Tetrahydrofuran aufgenommen. Die Suspension wird zentrifugiert und der Überstand im Vakuum eingeengt. Der Rückstand wird in Tetrahydrofuran aufgenommen und mit äquimolarer Menge konzentrierter Salzsäure versetzt. Nach Zugabe von Ether kristallisiert das Verfahrensprodukt (DL-Form) als Hydrochlorid bei -20° C aus. Schmelzpunkt 136-138° C - (Zersetzung).
$R_f$-Wert: 0,83 (stationäre Phase Cellulose).

Beispiel 9

2-{2-[N,N'-Bis-(2-chlorethyl)-diamido-phosphoryl-oxy]ethyl}-thiazolidin-4-carbonsäure

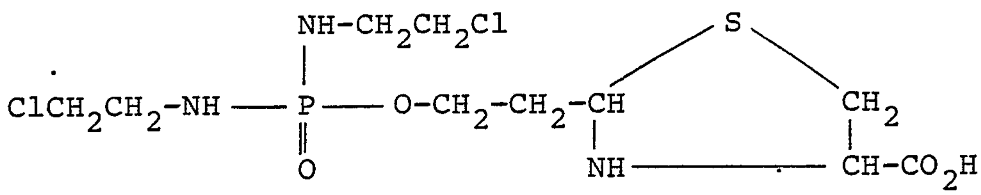

4,2 g (12 mMol) N,N'-Bis-(2-chlorethyl)-phosphorsäurediamid-3,3-diethoxy-propylester werden mit der 1,5fach molaren Menge L-Cystein in 100 ml 0,01 N Ameisensäure inkubiert (1 Stunde bei 60° C).
Das Reaktionsgemisch wird mit Chloroform ausgeschüttelt und am Gefriertrockner eingeengt.
Das farblose Pulver, das zu etwa gleichen Teilen aus der Thiazolidin-Verbindung und L-Cystein besteht, wird mit Peroxid-freiem Tetrahydrofuran extrahiert (ca. 100 ml Tetrahydrofuran/1 g Thiazolidin-Verbindung).
Nach Abzentrifugieren des Rückstandes und Einengen der Tetrahydrofuran-Lösung bleibt die Thiazolidin-Verbindung als farbloses Pulver zurück.
Dünnschichtchromatographie auf Cellulose-Platte
Laufmittel: $CH_3CN/H_2O$ 2:1
$R_f$ = 0,86

Die Ausgangssubstanz N,N'-Bis-(2-Chlorethyl)-phosphorsäurediamid-3,3-diethoxy-propylester kann beispielsweise wie folgt erhalten werden:
In einer Lösung von 34,5 g (130 mMol) frisch hergestelltem Phosphorsäure-3,3-diethoxy-propylester-dichlorid in 120 ml trockenem $CH_2Cl_2$ wird die doppelt molare Menge Chlorethylamin-Hydrochlorid suspendiert. Bei 0° C tropft man 4 Äquivalente (bezogen auf ein Mol Phosphorsäure-3,3-diethoxy-propylester-dichlorid) Triethylamin in $CH_2Cl_2$ zu und läßt 16 Stunden bei ca. 5° C rühren.
Aus ausgefallene Hydrochlorid wird durch Schütteln mit Eiswasser entfernt, die organische Phase über Natriumsulfat getrocknet und mit Aktivkohle ausgeschüttelt.
Zur weiteren Reinigung kann das Produkt über eine kurze Kieselgel-Säule gegeben werden: Laufmittel Methylenchlorid.

Beispiel 10

2-{2-[N,N'-Bis (2-chlorethyl)-diamido-phosphoryl-oxy]ethyl}1,5-perhydrothiazin(homothiazolidin)-4-carbonsäure

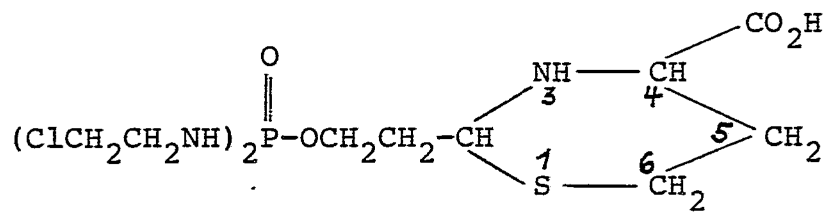

4,2 g (12 mMol) N,N'-Bis-(2-chlorethyl)-phosphorsäurediamid-1,3-diethoxy-propylester werden mit der 1,5fach molaren Menge DL-Homocystein in 100 ml 0,01 N Ameisensäure inkubiert (1 Stunde bei 60° C). Das Reaktionsgemisch wird mit Chloroform ausgeschüttelt und am Gefriertrockner eingeengt.
Das farblose Pulver, das zu etwa gleichen Teilen aus der Perhydrothiazinyl-Verbindung und DL-Homocystein besteht, wird mit Peroxid-freiem Tetrahydrofuran extrahiert (ca. 100 ml Tetrahydrofuran/1 g Reaktionsprodukt). Nach Abzentrifugieren des Rückstandes und Einengen der Tetrahydrofuran-Lösung bleibt die Perhydrothiazinyl-Verbindung als farbloses Pulver zurück.

Dünnschichtchromatographie auf Cellulose-Platte
Laufmittel: $CH_3CN/H_2O$ 2:1
$R_f$ = 0,86

Beispiele für pharmazeutische Zubereitungen

Lyophilisat der Verbindung gemäß Beispiel 1

100 g Verbindung gemäß Beispiel 1 und 160 g Mannit werden unter Stickstoffbegasung und Lichtausschluß in 4 Liter Wasser für Injektionszwecke gelöst. Die Lösung wird durch ein geeignetes Membranfilter sterilfiltriert und unter aseptischen Bedingungen in Anteilen von 20 ml in 50 ml Injektionsflaschen abgefüllt. Die Injektionsflaschen werden mit Gefriertrocknungsstopfen versehen, der Inhalt gefriergetrocknet. Anschließend wird die Gefriertrocknungsanlage mit trockenem Stickstoff belüftet und die Injektionsflaschen in der Anlage verschlossen.
Der Restwassergehalt des Lyophilisats darf 0,5 % nicht übersteigen. Zur Herstellung der Injektionslösung wird der Inhalt der Flaschen in 20 ml Wasser für Injektionszwecke gelöst. Die Injektionslösung ist isoton. 1 ml Injektionslösung enthält 25 mg Wirkstoff.

Lyophilisat der Verbindung gemäß Beispiel 1 mit 5 Mol% Cystein

100 g Verbindung gemäß Beispiel 1, 1,6 g Cystein und 160 g Mannit werden unter Stickstoffbegasung und Lichtausschluß in 4 Liter Wasser für Injektionszwecke gelöst. Die Lösung wird durch ein geeignetes Membranfilter sterilfiltriert und unter aseptischen Bedingungen in Anteilen von 20 ml in 50 ml Injektionsflaschen abgefüllt. Die Injektionsflaschen werden mit Gefriertrocknungsstopfen versehen, der Inhalt gefriergetrocknet. Anschließend wird die Gefriertrocknungsanlage mit trockenem Stickstoff belüftet und die Injektionsflaschen in der Anlage verschlossen. Der Restwassergehalt des Lyophilisats darf 0,5 % nicht übersteigen. Zur Herstellung der Injektionslösung wird der Inhalt der Flaschen in 20 ml Wasser für Injektionszwecke gelöst. Die Injektionslösung ist isoton. 1 ml Injektionslösung enthält 25 mg Wirkstoff und 0,4 mg Cystein.

## Ansprüche

1. Verbindungen der allgemeinen Formel

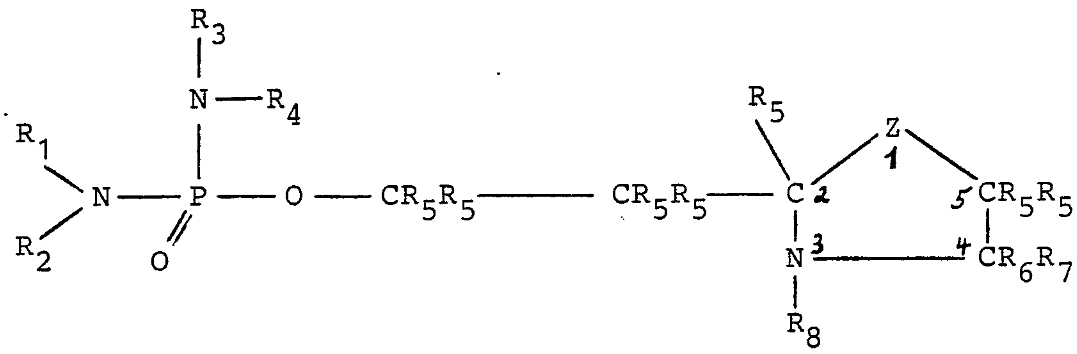

I

worin die Reste $R_1$, $R_2$, $R_3$ und $R_4$ gleich oder verschieden sind und Wasserstoff, $C_1$-$C_4$-Alkyl, 2-Chlorethyl, 2-Bromethyl oder 2-$C_1$-$C_4$-Alkansulfonyloxyethyl bedeuten und wobei mindestens zwei dieser Reste 2-Chlorethyl, 2-Bromethyl oder 2-$C_1$-$C_4$-Alkansulfonyloxyethyl bedeuten, $R_8$ Wasserstoff, $C_1$-$C_6$-Alkyl, $C_1$-$C_6$-Alkoxycarbonyl, $C_2$-$C_6$-Alkanoyl, der Rest einer natürlichen Aminosäure oder eines natürlichen Dipeptids, wobei eine freie Carboxygruppe auch durch $C_1$-$C_6$-Alkyl verestert sein kann, bedeutet, oder worin $R_8$ Aminocarbonyl oder Aminocarbonyl mit einem oder zwei $C_1$-$C_6$-Alkylresten am Stickstoffatom ist, die Reste $R_6$ und $R_7$ Wasserstoff sind oder zusammen ein Sauerstoffatom bilden oder worin $R_6$ Wasserstoff ist, und in diesem Falle $R_7$ eine Carboxylgruppe, eine $C_1$-$C_6$-Alkoxycarbonylgruppe, eine Aminocarbonylgruppe, eine $C_1$-$C_6$-Alkylaminocarbonylgruppe, eine Di-$C_1$-$C_6$-Alkylaminocarbonylgruppe, oder eine Carbonsäureamidgruppe ist, wobei der Amidteil den Rest einer natürlichen Aminosäure oder eines natürlichen Dipeptids oder deren $C_1$-$C_6$-Alkylester darstellt, Z ein Schwefelatom, ein Sauerstoffatom, die Gruppe $>NR_5$, die Gruppe $-S-C(R_5)_2-$, $-O-C(R_5)_2-$oder $-NR_5-C(R_5)_2-$bedeutet, und die Reste $R_5$ gleich oder verschieden sind und Wasserstoff oder $C_1$-$C_6$-Alkyl darstellen, und deren Salze mit physiologisch verträglichen Säuren oder Kationen.

2. Verfahren zur Herstellung von Verbindungen der allgemeinen Formel

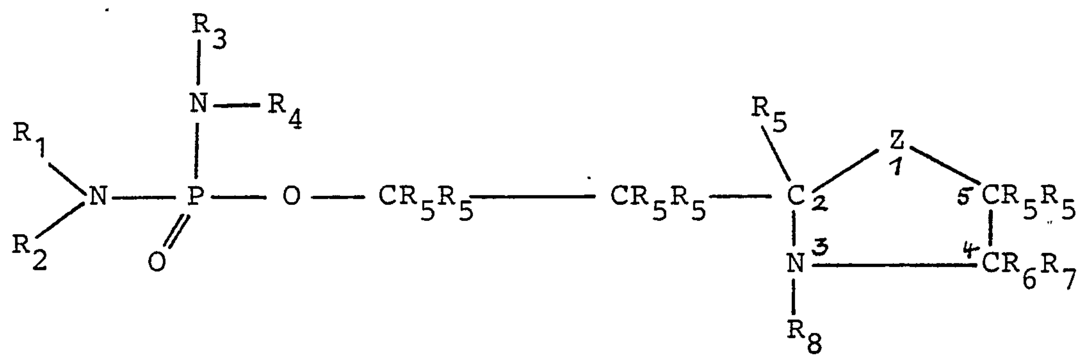

I

worin die Reste $R_1$, $R_2$, $R_3$ und $R_4$ gleich oder verschieden sind und Wasserstoff, $C_1$-$C_4$-Alkyl, 2-Chlorethyl, 2-Bromethyl oder 2-$C_1$-$C_4$-Alkansulfonyloxyethyl bedeuten und wobei mindestens zwei dieser Reste 2-Chlorethyl, 2-Bromethyl oder 2-$C_1$-$C_4$-Alkansulfonyloxyethyl bedeuten, $R_8$ Wasserstoff, $C_1$-$C_6$-Alkyl, $C_1$-$C_6$-Alkoxycarbonyl, $C_2$-$C_6$-Alkanoyl, der Rest einer natürlichen Aminosäure oder eines natürlichen Dipeptids, wobei eine freie Carboxygruppe auch durch $C_1$-$C_6$-Alkyl verestert sein kann, bedeutet, oder worin $R_8$ Aminocarbonyl oder Aminocarbonyl mit einem oder zwei $C_1$-$C_6$-Alkylresten am Stickstoffatom ist, die Reste $R_6$ und $R_7$ Wasserstoff sind oder zusammen ein Sauerstoffatom bilden oder worin $R_6$ Wasserstoff ist, und in diesem Falle $R_7$ eine Carboxylgruppe, eine $C_1$-$C_6$-Alkoxycarbonylgruppe, eine Aminocarbonylgruppe, eine $C_1$-$C_6$-Alkylaminocarbonylgruppe, eine Di-$C_1$-$C_6$-Alkylaminocarbonylgruppe, oder eine Carbonsäureamidgruppe ist, wobei der Amidteil den Rest einer natürlichen Aminosäure oder eines natürlichen Dipeptids oder deren $C_1$-$C_6$-Alkylester darstellt, Z ein Schwefelatom, ein Sauerstoffatom, die Gruppe $>NR_5$, die Gruppe $-S-C(R_5)_2-$, $-O-C(R_5)_2-$oder $-NR_5-C(R_5)_2-$bedeutet, und die Reste $R_5$ gleich oder verschieden sind und Wasserstoff oder $C_1$-$C_6$-Alkyl darstellen, und deren Salze mit physiologisch verträglichen Säuren oder Kationen,

dadurch gekennzeichnet,

daß man eine Verbindung der allgemeinen Formel

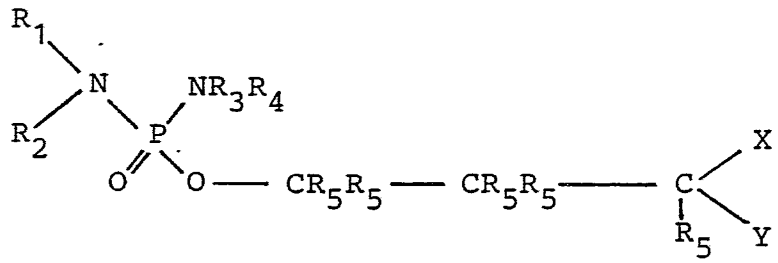

II

worin $R_1$, $R_2$, $R_3$, $R_4$ und $R_5$ die angegebenen Bedeutungen haben und entweder X und Y je eine $C_1$-$C_6$-Alkoxy-oder $C_1$-$C_6$-Alkylthiogruppe oder zusammen ein Sauerstoffatom, einen gesättigten Alkylendioxyring aus 1 bis 5 C-Atomen, einen gesättigten Alkylendithioring aus 1 bis 5 C-Atomen oder eine Hydroxyiminogruppe darstellen, oder worin X zusammen mit dem Rest $R_4$ eine einfache Bindung unter Bildung des 6-Ringes darstellt und in diesem Falle Y eine Hydroxygruppe, eine $C_1$-$C_6$-Alkoxygruppe oder die Gruppe S-alk-$SO_3Z$ ist, wobei alk eine $C_2$-$C_6$-Alkylenbrücke darstellt und Z Wasserstoff oder ein Kation ist, oder worin Y eine Benzylthiogruppe, eine $C_1$-$C_6$-Alkanoylthiogruppe oder eine gegebenenfalls durch eine Carboxygruppe, eine Hydroxygruppe oder $C_1$-$C_6$-Carbalkoxygruppe substituierte $C_1$-$C_{10}$-Alkylthiogruppe ist, mit einer Verbindung der allgemeinen Formel

HZ-$CR_5R_5$-$CR_6R_7$-$NHR_8$ III

umsetzt, wobei Z, $R_5$, $R_6$, $R_7$ und $R_8$ die angegebenen Bedeutungen haben,
und gegebenenfalls in eine Verbindung der Formel I, worin $R_8$ Wasserstoff ist, und die übrigen Symbole die angegebenen Bedeutungen haben, durch Alkylierung oder Acylierung den Rest $R_8$ einführt, wobei $R_8$ die angegebenen Bedeutungen außer Wasserstoff hat, gegebenenfalls den Rest $R_8$ abspaltet, gegebenenfalls in Z, falls Z die Gruppe $\geq$ NH oder -NH-C$(R_5)_2$-ist, durch Alkylierung eine $C_1$-$C_6$-Alkylgruppe einführt, sowie gegebenenfalls in einer Verbindung der Formel I den Rest $R_7$, falls dieser kein Wasserstoffatom ist, in eine andere hierfür mögliche Bedeutung überführt, und die erhaltenen Verbindungen gegebenenfalls in die Salze mit physiologisch verträglichen Kationen oder Anionen überführt.

3. Verbindungen der Formel I zur Anwendung als therapeutische Wirkstoffe.

4. Arzneimittel, enthaltend eine Verbindung der allgemeinen Formel I neben üblichen Träger-und/oder Verdünnungs-beziehungsweise Hilfsstoffen

5. Verfahren zur Herstellung eines Arzneimittels,
dadurch gekennzeichnet,
daß eine Verbindung der allgemeinen Formel I mit gebräuchlichen pharmazeutischen Trägerstoffen und/oder Verdünnungsmitteln beziehungsweise sonstigen Hilfsstoffen zu pharmazeutischen Zubereitungen verarbeitet beziehungsweise in eine therapeutisch anwendbare Form gebracht wird.

6. Verwendung von Verbindungen der allgemeinen Formel I zur Herstellung von Arzneimitteln.

Patentansprüche für folgenden Vertragsstaat: AT

1. Verfahren zur Herstellung von Verbindungen der allgemeinen Formel

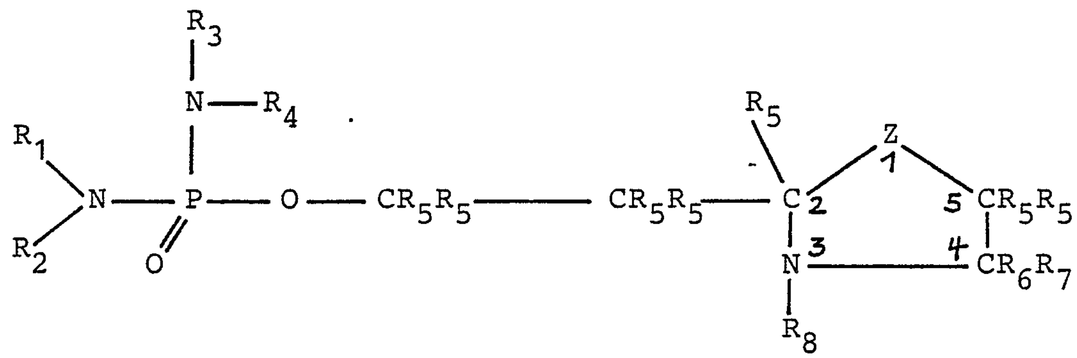

I

worin die Reste $R_1$, $R_2$, $R_3$ und $R_4$ gleich oder verschieden sind und Wasserstoff, $C_1$-$C_4$-Alkyl, 2-Chlorethyl, 2-Bromethyl oder 2-$C_1$-$C_4$-Alkansulfonyloxyethyl bedeuten und wobei mindestens zwei dieser Reste 2-Chlorethyl, 2-Bromethyl oder 2-$C_1$-$C_4$-Alkansulfonyloxyethyl bedeuten, $R_8$ Wasserstoff, $C_1$-$C_6$-Alkyl, $C_1$-$C_6$-Alkoxycarbonyl, $C_2$-$C_6$-Alkanoyl, der Rest einer natürlichen Aminosäure oder eines natürlichen Dipeptids, wobei eine freie Carboxygruppe auch durch $C_1$-$C_6$-Alkyl verestert sein kann, bedeutet, oder worin $R_8$ Aminocarbonyl oder Aminocarbonyl mit einem oder zwei $C_1$-$C_6$-Alkylresten am Stickstoffatom ist, die Reste $R_6$ und $R_7$ Wasserstoff sind oder zusammen ein Sauerstoffatom bilden oder worin $R_6$ Wasserstoff ist, und in diesem Falle $R_7$ eine Carboxylgruppe, eine $C_1$-$C_6$-Alkoxycarbonylgruppe, eine Aminocarbonylgruppe, eine $C_1$-$C_6$-Alkylaminocarbonylgruppe, eine Di-$C_1$-$C_6$-Alkylaminocarbonylgruppe, oder eine Carbonsäureamidgruppe ist, wobei der Amidteil den Rest einer natürlichen Aminosäure oder eines natürlichen Dipeptids oder deren $C_1$-$C_6$-Alkylester darstellt, Z ein Schwefelatom, ein Sauerstoffatom, die Gruppe $\gtrdot NR_5$, die Gruppe $-S-C(R_5)_2-$, $-O-C(R_5)_2-$oder $-NR_5-C(R_5)_2-$bedeutet,
und die Reste $R_5$ gleich oder verschieden sind und Wasserstoff oder $C_1$-$C_6$-Alkyl darstellen, und deren Salze mit physiologisch verträglichen Säuren oder Kationen,
dadurch gekennzeichnet,
daß man eine Verbindung der allgemeinen Formel

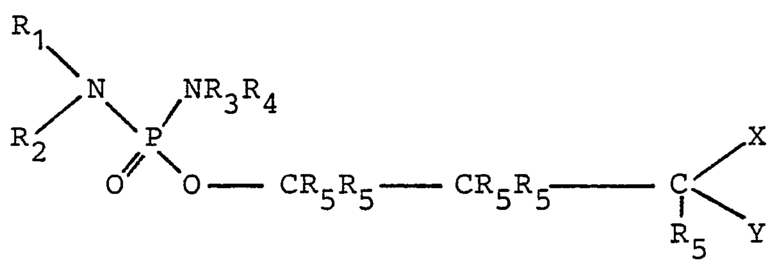

II

worin $R_1$, $R_2$, $R_3$, $R_4$ und $R_5$ die angegebenen Bedeutungen haben und entweder X und Y je eine $C_1$-$C_6$-Alkoxy-oder $C_1$-$C_6$-Alkylthiogruppe oder zusammen ein Sauerstoffatom, einen gesättigten Alkylendioxyring aus 1 bis 5 C-Atomen, einen gesättigten Alkylendithioring aus 1 bis 5 C-Atomen oder eine Hydroxyiminogruppe darstellen, oder worin X zusammen mit dem Rest $R_4$ eine einfache Bindung unter Bildung des 6-Ringes darstellt und in diesem Falle Y eine Hydroxygruppe, eine $C_1$-$C_6$-Alkoxygruppe oder die Gruppe S-alk-$SO_3Z$ ist, wobei alk eine $C_2$-$C_6$-Alkylenbrücke darstellt und Z Wasserstoff oder ein Kation ist, oder worin Y eine Benzylthiogruppe, eine $C_1$-$C_6$-Alkanoylthiogruppe oder eine gegebenenfalls durch eine Carboxygruppe, eine Hydroxygruppe oder $C_1$-$C_6$-Carbalkoxygruppe substituierte $C_1$-$C_{10}$-Alkylthiogruppe ist, mit einer Verbindung der allgemeinen Formel

$HZ-CR_5R_5-CR_6R_7-NHR_8$ III

umsetzt, wobei Z, $R_5$, $R_6$, $R_7$ und $R_8$ die angegebenen Bedeutungen haben,
und gegebenenfalls in eine Verbindung der Formel I, worin $R_8$ Wasserstoff ist, und die übrigen Symbole die angegebenen Bedeutungen haben, durch Alkylierung oder Acylierung den Rest $R_8$ einführt, wobei $R_8$ die angegebenen Bedeutungen außer Wasserstoff hat, gegebenenfalls den Rest $R_8$ abspaltet, gegebenenfalls in Z, falls Z die Gruppe $\gtrdot$ NH oder $-NH-C(R_5)_2-$ist, durch Alkylierung eine $C_1$-$C_6$-Alkylgruppe einführt, sowie gegebenenfalls in einer Verbindung der Formel I den Rest $R_7$, falls dieser kein Wasserstoffatom ist, in eine

andere hierfür mögliche Bedeutung überführt, und die erhaltenen Verbindungen gegebenenfalls in die Salze mit physiologisch verträglichen Kationen oder Anionen überführt.

2. Verfahren zur Herstellung eines (antitumor-wirksamen und/oder gegen AIDS wirksamen) Arzneimittels,
dadurch gekennzeichnet,
daß man Verbindungen der Formel I, worin die Reste $R_1$ bis $R_8$ und Z die in Anspruch 1 angegebenen Bedeutungen haben, beziehungsweise deren Salze mit physiologisch unbedenklichen Säuren oder Kationen zusammen mit üblichen Träger-und/oder Verdünnungs-beziehungsweise Hilfsstoffen bei Temperaturen zwischen 20 und 100° C vermischt beziehungsweise homogenisiert, die so erhaltenen Mischung, gegebenenfalls nach Zusatz von 0,02 -0,05 Mol (bezogen auf 1 Mol Verbindung I) Thiol-Verbindung mit freier SH-Gruppe, zur Herstellung von Zubereitungen, die in der Dosierungseinheit 50 bis 3000 mg Wirkstoff (als Antitumormittel) oder 0,05 bis 500 mg Wirkstoff der Formel I (als Mittel gegen AIDS) der Formel I enthalten, lyophilisiert oder in Hohlzellen entsprechender Größe ausgießt oder in Kapseln entsprechender Größe abfüllt oder granuliert und dann gegebenenfalls unter Zusatz von weiteren üblichen Hilfsstoffen zu Tabletten verpreßt.

3. Verfahren zur Herstellung eines (antitumor-wirksamen und/oder gegen AIDS wirksamen) Arzneimittels,
dadurch gekennzeichnet,
daß man Verbindungen der Formel I, worin die Reste $R_1$ bis $R_8$ und Z die in Anspruch 1 angegebene Bedeutung haben, beziehungsweise deren Salze mit physiologisch unbedenklichen Säuren oder Kationen, gegebenenfalls nach Zusatz von 0,02 -0,05 Mol (bezogen auf 1 Mol Verbindung I) einer Thiol-Verbindung mit freier SH-Gruppe, mit einem oder mehreren der folgenden Stoffe: Stärke, Mannit, Cellulose, Lactose, Formalin-Casein, modifizierte Stärke, Magnesiumstearat, Calciumhydrogenphosphat, hochdisperse Kieselsäure, Talkum vermischt, die erhaltene Mischung, gegebenenfalls mit einer wässrigen Lösung, die als Bestandteil mindestens Gelatine, Stärke, Polyvinylpyrrolidon, Vinylpyrrolidon-Vinylacetat-Copolymerisat und/oder Polyoxyethylensorbitmonooleat enthält, granuliert, das Granulat gegebenenfalls mit einem oder mehreren der obengenannten Hilfsstoffe homogenisiert, und diese Mischung zu Tabletten verpresst oder in Kapseln abfüllt, wobei die Tabletten oder Kapseln in der Dosierungseinheit jeweils 50 bis 3000 mg Wirkstoff der Formel I (als Antitumormittel) oder 0,05 bis 500 mg Wirkstoff der formel I (als Mittel gegen AIDS) enthalten.

4. Verfahren zur Herstellung eines (antitumor-wirksamen und/oder gegen AIDS wirksamen) Arzneimittels,
dadurch gekennzeichnet,
daß man Verbindungen der Formel I oder deren Salze mit physiologisch unbedenklichen Säuren oder Kationen, gegebenenfalls nach Zusatz von 0,02 -0,05 Mol (bezogen auf 1 Mol Verbindung I) Thiol-Verbindung mit freier SH-Gruppe, nach Zusatz von Sojalecithin bei Temperaturen zwischen 33 -37° C in geschmolzenem Hartfett suspendiert und homogenisiert und anschließend die Mischung in Hohlzellen ausgießt, wobei die Dosierungseinheit 50 bis 3000 mg Wirkstoff der Formel I (als Antitumormittel) oder 0,05 bis 500 mg Wirkstoff der Formel I (als Mittel gegen AIDS) enthält.

5. Verfahren zur Herstellung einer (antitumor-wirksamen und/oder gegen AIDS wirksamen) Injektionslösung,
dadurch gekennzeichnet,
daß man Verbindungen der Formel I oder deren Salze mit physiologisch unbedenklichen Säuren oder Kationen, gegebenenfalls nach Zusatz von 0,02 -0,05 Mol (bezogen auf 1 Mol Verbindung I) einer Thiol-Verbindung mit freier SH-Gruppe, sowie gegebenenfalls nach Zusatz von Mannit, in Wasser oder Pflanzenöl, gegebenenfalls in Gegenwart eines üblichen Emulgators, bei Temperaturen zwischen 15 -50° C auflöst, und die so erhaltene Lösung mit soviel Wasser oder Pflanzenöl auffüllt, daß die Endlösung 0,01 bis 5 Gewichtsprozent an Wirkstoff der Formel I enthält.

6. Verwendung von Verbindungen der allgemeinen Formel I, worin die Reste $R_1$ bis $R_8$ und Z die in Anspruch 1 angegebene Bedeutung haben, zur Herstellung von Arzneimitteln.

Patentansprüche für folgende Vertragsstaaten: GR, ES

1. Verfahren zur Herstellung von Verbindungen der allgemeinen Formel

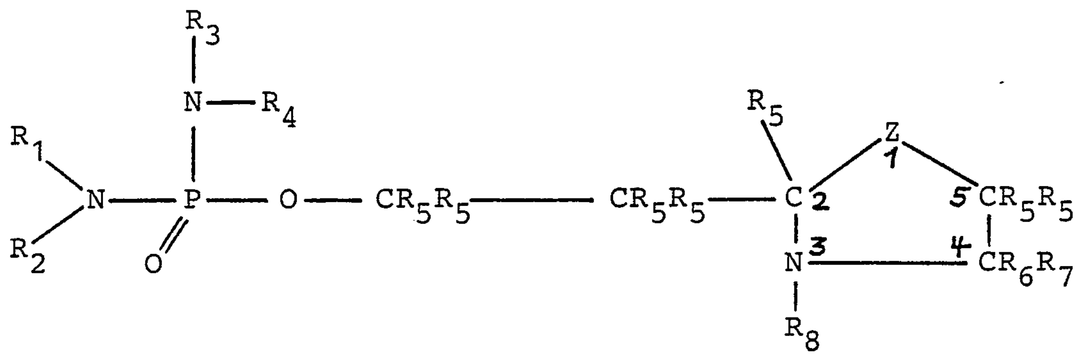

I

worin die Reste $R_1$, $R_2$, $R_3$ und $R_4$ gleich oder verschieden sind und Wasserstoff, $C_1$-$C_4$-Alkyl, 2-Chlorethyl, 2-Bromethyl oder 2-$C_1$-$C_4$-Alkansulfonyloxyethyl bedeuten und wobei mindestens zwei dieser Reste 2-Chlorethyl, 2-Bromethyl oder 2-$C_1$-$C_4$-Alkansulfonyloxyethyl bedeuten, $R_8$ Wasserstoff, $C_1$-$C_6$-Alkyl, $C_1$-$C_6$-Alkoxycarbonyl, $C_2$-$C_6$-Alkanoyl, der Rest einer natürlichen Aminosäure oder eines natürlichen Dipeptids, wobei eine freie Carboxygruppe auch durch $C_1$-$C_6$-Alkyl verestert sein kann, bedeutet, oder worin $R_8$ Aminocarbonyl oder Aminocarbonyl mit einem oder zwei $C_1$-$C_6$-Alkylresten am Stickstoffatom ist, die Reste $R_6$ und $R_7$ Wasserstoff sind oder zusammen ein Sauerstoffatom bilden oder worin $R_6$ Wasserstoff ist, und in diesem Falle $R_7$ eine Carboxylgruppe, eine $C_1$-$C_6$-Alkoxycarbonylgruppe, eine Aminocarbonylgruppe, eine $C_1$-$C_6$-Alkylaminocarbonylgruppe, eine Di-$C_1$-$C_6$-Alkylaminocarbonylgruppe, oder eine Carbonsäureamidgruppe ist, wobei der Amidteil den Rest einer natürlichen Aminosäure oder eines natürlichen Dipeptids oder deren $C_1$-$C_6$-Alkylester darstellt, Z ein Schwefelatom, ein Sauerstoffatom, die Gruppe $>NR_5$, die Gruppe $-S-C(R_5)_2-$, $-O-C(R_5)_2-$oder $-NR_5-C(R_5)_2-$bedeutet,
und die Reste $R_5$ gleich oder verschieden sind und Wasserstoff oder $C_1$-$C_6$-Alkyl darstellen,
und deren Salze mit physiologisch verträglichen Säuren oder Kationen,
dadurch gekennzeichnet,
daß man eine Verbindung der allgemeinen Formel

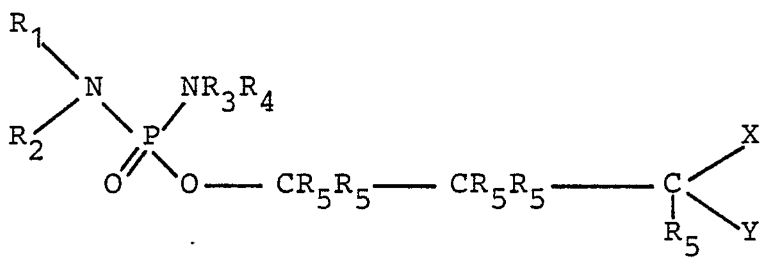

II

worin $R_1$, $R_2$, $R_3$, $R_4$ und $R_5$ die angegebenen Bedeutungen haben und entweder X und Y je eine $C_1$-$C_6$-Alkoxy-oder $C_1$-$C_6$-Alkylthiogruppe oder zusammen ein Sauerstoffatom, einen gesättigten Alkylendioxyring aus 1 bis 5 C-Atomen, einen gesättigten Alkylendithioring aus 1 bis 5 C-Atomen oder eine Hydroxyiminogruppe darstellen, oder worin X zusammen mit dem Rest $R_4$ eine einfache Bindung unter Bildung des 6-Ringes darstellt und in diesem Falle Y eine Hydroxygruppe, eine $C_1$-$C_6$-Alkoxygruppe oder die Gruppe S-alk-$SO_3Z$ ist, wobei alk eine $C_2$-$C_6$-Alkylenbrücke darstellt und Z Wasserstoff oder ein Kation ist, oder worin Y eine Benzylthiogruppe, eine $C_1$-$C_6$-Alkanoylthiogruppe oder eine gegebenenfalls durch eine Carboxygruppe, eine Hydroxygruppe oder $C_1$-$C_6$-Carbalkoxygruppe substituierte $C_1$-$C_{10}$-Alkylthiogruppe ist, mit einer Verbindung der allgemeinen Formel

$HZ-CR_5R_5-CR_6R_7-NHR_8$ III

umsetzt, wobei Z, $R_5$, $R_6$, $R_7$ und $R_8$ die angegebenen Bedeutungen haben,
und gegebenenfalls in eine Verbindung der Formel I, worin $R_8$ Wasserstoff ist, und die übrigen Symbole die angegebenen Bedeutungen haben, durch Alkylierung oder Acylierung den Rest $R_8$ einführt, wobei $R_8$ die angegebenen Bedeutungen außer Wasserstoff hat, gegebenenfalls den Rest $R_8$ abspaltet, gegebenenfalls in Z, falls Z die Gruppe $>NH$ oder $-NH-C(R_5)_2-$ist, durch Alkylierung eine $C_1$-$C_6$-Alkylgruppe einführt, sowie gegebenenfalls in einer Verbindung der Formel I den Rest $R_7$, falls dieser kein Wasserstoffatom ist, in eine

andere hierfür mögliche Bedeutung überführt, und die erhaltenen Verbindungen gegebenenfalls in die Salze mit physiologisch verträglichen Kationen oder Anionen überführt.

2. Verfahren zur Herstellung eines Arzneimittels, enthaltend als Wirkstoff mindestens eine Verbindung der allgemeinen Formel I, worin die Reste $R_1$ bis $R_8$ und Z die in Anspruch 1 angegebene Bedeutung haben,
dadurch gekennzeichnet,
daß mindestens eine Verbindung der Formel I mit gebräuchlichen pharmazeutischen Trägerstoffen und/oder Verdünnungsmitteln beziehungsweise sonstigen Hilfsstoffen zu pharmazeutischen Zubereitungen verarbeitet beziehungsweise in eine therapeutisch anwendbare Form gebracht wird.

3. Verfahren zur Herstellung eines (antitumor-wirksamen und/oder gegen AIDS wirksamen) Arzneimittels,
dadurch gekennzeichnet,
daß man Verbindungen der Formel I, worin die Reste $R_1$ bis $R_8$ und Z die in Anspruch 1 angegebenen Bedeutungen haben, beziehungsweise deren Salze mit physiologisch unbedenklichen Säuren oder Kationen zusammen mit üblichen Träger-und/oder Verdünnungs-beziehungsweise Hilfsstoffen bei Temperaturen zwischen 20 und 100° C vermischt beziehungsweise homogenisiert, die so erhaltene Mischung, gegebenenfalls nach Zusatz von 0,02 -0,05 Mol (bezogen auf 1 Mol Verbindung I) Thiol-Verbindung mit freier SH-Gruppe, zur Herstellung von Zubereitungen, die in der Dosierungseinheit 50 bis 3000 mg Wirkstoff (als Antitumormittel) oder 0,05 bis 500 mg Wirkstoff der Formel I (als Mittel gegen AIDS) der Formel I enthalten, lyophilisiert oder in Hohlzellen entsprechender Größe ausgießt oder in Kapseln entsprechender Größe abfüllt oder granuliert und dann gegebenenfalls unter Zusatz von weiteren üblichen Hilfsstoffen zu Tabletten verpreßt.

4. Verfahren zur Herstellung eines (antitumor-wirksamen und/oder gegen AIDS wirksamen) Arzneimittels,
dadurch gekennzeichnet,
daß man Verbindungen der Formel I, worin die Reste R bis $R_8$ und Z die in Anspruch 1 angegebene Bedeutung haben, beziehungsweise deren Salze mit physiologisch unbedenklichen Säuren oder Kationen, gegebenenfalls nach Zusatz von 0,02 -0,05 Mol (bezogen auf 1 Mol Verbindung I) einer Thiol-Verbindung mit freier SH-Gruppe, mit einem oder mehreren der folgenden Stoffe: Stärke, Mannit, Cellulose, Lactose, Formalin-Casein, modifizierte Stärke, Magnesiumstearat, Calciumhydrogenphosphat, hochdisperse Kieselsäure, Talkum vermischt, die erhaltene Mischung, gegebenenfalls mit einer wässrigen Lösung, die als Bestandteil mindestens Gelatine, Stärke, Polyvinylpyrrolidon, Vinylpyrrolidon-Vinylacetat-Copolymerisat und/oder Polyoxyethylensorbitmonooleat enthält, granuliert, das Granulat gegebenenfalls mit einem oder mehreren der obengenannten Hilfsstoffe homogenisiert, und diese Mischung zu Tabletten verpresst oder in Kapseln abfüllt, wobei die Tabletten oder Kapseln in der Dosierungseinheit jeweils 50 bis 3000 mg Wirkstoff der Formel I (als Antitumormittel) oder 0,05 bis 500 mg Wirkstoff der Formel I (als Mittel gegen AIDS) enthalten.

5. Verfahren zur Herstellung eines (antitumor-wirksamen und/oder gegen AIDS wirksamen) Arzneimittels,
dadurch gekennzeichnet,
daß man Verbindungen der Formel I oder deren Salze mit physiologisch unbedenklichen Säuren oder Kationen, gegebenenfalls nach Zusatz von 0,02 -0,05 Mol (bezogen auf 1 Mol Verbindung I) Thiol-Verbindung mit freier SH-Gruppe, nach Zusatz von Sojalecithin bei Temperaturen zwischen 33 -37° C in geschmolzenem Hartfett suspendiert und homogenisiert und anschließend die Mischung in Hohlzellen ausgießt, wobei die Dosierungseinheit 50 bis 3000 mg Wirkstoff der Formel I (als Antitumormittel) oder 0,05 bis 500 mg Wirkstoff der Formel I (als Mittel gegen AIDS) enthält.

6. Verfahren zur Herstellung einer (antitumor-wirksamen und/oder gegen AIDS wirksamen) Injektionslösung,
dadurch gekennzeichnet,
daß man Verbindungen der Formel I oder deren Salze mit physiologisch unbedenklichen Säuren oder Kationen, gegebenenfalls nach Zusatz von 0,02 -0,05 Mol (bezogen auf 1 Mol Verbindung I) einer Thiol-Verbindung mit freier SH-Gruppe, sowie gegebenenfalls nach Zusatz von Mannit, in Wasser oder Pflanzenöl, gegebenenfalls in Gegenwart eines üblichen Emulgators, bei Temperaturen zwischen 15 -50° C auflöst, und die so erhaltene Lösung mit soviel Wasser oder Pflanzenöl auffüllt, daß die Endlösung 0,01 bis 5 Gewichtsprozent an Wirkstoff der Formel I enthält.

7. Verwendung von Verbindungen der allgemeinen Formel I, worin die Reste $R_1$ bis $R_8$ und Z die in Anspruch 1 angegebene Bedeutung haben, zur Herstellung von Arzneimitteln.